(19) Europäisches Patentamt
European Patent Office
Office européen des brevets

(11) EP 3 126 392 B1

# (12) EUROPEAN PATENT SPECIFICATION

(45) Date of publication and mention of the grant of the patent:
**11.09.2019 Bulletin 2019/37**

(21) Application number: **15714136.7**

(22) Date of filing: **19.03.2015**

(51) Int Cl.:
***C07K 16/28*** *(2006.01)* ***A61P 17/06*** *(2006.01)*

(86) International application number:
**PCT/US2015/021613**

(87) International publication number:
**WO 2015/153144 (08.10.2015 Gazette 2015/40)**

## (54) METHODS OF TREATING NAIL AND SCALP PSORIASIS

VERFAHREN ZUR BEHANDLUNG VON NAGEL- UND KOPFHAUTPSORIASIS

PROCÉDÉS DE TRAITEMENT DU PSORIASIS DES ONGLES ET DU CUIR CHEVELU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.03.2014 US 201461972638 P**
**31.07.2014 US 201462031850 P**
**26.08.2014 US 201462041879 P**

(43) Date of publication of application:
**08.02.2017 Bulletin 2017/06**

(73) Proprietor: **Kirin-Amgen, Inc.**
**Thousand Oaks, CA 91320-1799 (US)**

(72) Inventors:
- **NIRULA, Ajay**
  **San Diego, CA 92130 (US)**
- **NAKAGAWA, Hidemi**
  **Tokyo 105-8471 (JP)**
- **OHTAKI, Kenji**
  **Tokyo 100-8185 (JP)**
- **MATSUDO, Hiroki**
  **Tokyo 100-8185 (JP)**
- **KLEKOTKA, Paul**
  **Newbury Park, California 91320 (US)**
- **KRICORIAN, Gregory**
  **Thousand Oaks, California 91320-1799 (US)**

(74) Representative: **Carpmaels & Ransford LLP**
**One Southampton Row**
**London WC1B 5HA (GB)**

(56) References cited:
**WO-A1-2011/088120**

- **LEONARDI CRAIG ET AL: "Anti-Interleukin-17 Monoclonal Antibody Ixekizumab in Chronic Plaque Psoriasis", NEW ENGLAND JOURNAL OF MEDICINE, MASSACHUSETTS MEDICAL SOCIETY, US, vol. 366, no. 13, 29 March 2012 (2012-03-29), pages 1190-1199, XP002681513, ISSN: 0028-4793**
- **Anonymous: "NCT02052609 on 2014_01_31: ClinicalTrials.gov Archive", , 31 January 2014 (2014-01-31), XP055189143, Retrieved from the Internet: URL:https://clinicaltrials.gov/archive/NCT02052609/2014_01_31 [retrieved on 2015-05-13]**
- **KIM A PAPP ET AL: "Brodalumab, an Anti-Interleukin-17-Receptor Antibody for Psoriasis", NEW ENGLAND JOURNAL OF MEDICINE, MASSACHUSETTS MEDICAL SOCIETY, US, vol. 366, 29 March 2012 (2012-03-29), pages 1181-1189, XP002726554, ISSN: 0028-4793, DOI: 10.1056/NEJMOA1109017**
- **Y Umezawa: "BSD Medical Reports Clinical Study Data; brodalumab in psoriasis", Wireless News, 12 October 2014 (2014-10-12), XP055189076, Jacksonville Retrieved from the Internet: URL:http://search.proquest.com/docview/210289466 [retrieved on 2015-05-13]**
- **GUDJONSSON ET AL: "HLA-Cw6-Positive and HLA-Cw6-Negative Patients with Psoriasis Vulgaris have Distinct Clinical Features", , 1 February 2002 (2002-02-01), XP055218934, Retrieved from the Internet: URL:http://www.nature.com/jid/journal/v118/n2/pdf/5601404a.pdf [retrieved on 2015-10-07]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 126 392 B1

- **ARMSTRONG APRIL W ET AL: "Secukinumab in the Treatment of Palmoplantar, Nail, Scalp, and Pustular Psoriasis.", THE JOURNAL OF CLINICAL AND AESTHETIC DERMATOLOGY JUN 2016, vol. 9, no. 6 Suppl 1, June 2016 (2016-06) , pages S12-S16, ISSN: 1941-2789**

## Description

### FIELD OF THE INVENTION

The present invention relates to a therapeutic agent for nail psoriasis and scalp psoriasis comprising an IL-17 receptor A (IL 17RA or IL-17R) antigen binding proteins such as a monoclonal antibody or an antibody fragment thereof as an active ingredient. The present
invention relates to methods of treating nail and scalp psoriasis with IL-17RA antigen binding proteins, such as a monoclonal antibodies or antibody fragment thererof.

### BACKGROUND

Psoriasis is a chronic and debilitating immune-related inflammatory disease that can involve the scalp and nail beds. This disease causes significant social stigma for patients and also is a major economic burden. In some cases, nail or scalp psoriasis may be the only affected area and these patients are unlikely to meet the requirements for systemic or biologic therapies.

Psoriasis is thought to affect the scalp in two ways: psoriatic alopecia, without any scaliness, and regular scalp psoriasis, with typical epidermal involvement (Hermanns-Le et al., J. Biomed. Biotech. 2012: 1-6, 2012). Scalp psoriasis is very difficult to treat, and many times it is disabling for the patient as it causes many quality of life issues. Many topical therapies for scalp psoriasis are difficult and unpleasant to apply and as a result patient compliance and adherence to treatment regimens is often reduced. In addition, as the scalp qualifies as a small percentage of body surface area, many patients suffering from scalp psoriasis do not qualify for treatment with biological agents. The typical treatment scheme is topical, including tar and salicylic acid, followed by phototherapy, and then systemic therapies, such as methrotrexate and acitretin. The systemic therapies may cause further hair loss, and thereby exacerbate the hair loss associated with scalp psoriasis itself. (Kircik and Kumar, J. Drugs Dermolog. 9: s101-s105, 2010).

Nail psoriasis is often overlooked as the nails are largely asymptomatic in the early stages of disease. The current treaments for nail psoriasis are often poorly efficacious and associated with undesirable systemic effects. In addition, many of the treatments are time consuming and impractical for the patient. Due to the anatomical structure of the nail unit, it is generally difficult to achieve sufficient concentrations of adsorptive treatment agents in the involved nail, nail bed or matrix by topical administration. (Wozel, Clin. Derm. 26:448-459, 2008), while the small amount of surface area affected may not qualify a patient for systemic or biologic treatment.

IL-17A is an inflammatory cytokine initially identified as a transcript selectively expressed by activated T cells. IL-17RA is a ubiquitously expressed and shown to bind IL-17A with an affinity of approximately 0.5 nM (Yao et al., 1995, Immunity 3:811-821). Five additional IL-17-like ligands (IL-17B-IL-17F) and four additional IL-17RA-like receptors (IL-17RB-IL-17RE) have been identified (Kolls and Linden, 2004, Immunity 21:467-476).

IL-17A and IL-17F bind and activate IL-17RA. IL-17RA has been shown to be important in regulating immune responses. Activation of the IL-17RA leads to production of cytokines, chemokines, growth factors, and other proteins that contribute to the symptoms and/or pathology of numerous diseases. IL-17A is an inflammatory cytokine that induces the production of cytokines and other mediators leading to diseases and physiological effects such as inflammation, cartilage degradation, and bone resorption. IL-17Aalso plays a role in a number of inflammatory conditions including arthritis (rheumatoid arthritis), psoriasis, inflammatory bowel disease, multiple sclerosis, and asthma. (Li et al., 2004, Huazhong Univ. Sci. Technolog. Med. Sci. 24:294-296; Fujino et al., 2003, Gut. 52:65-70; Kauffman et al., 2004, J. Invest. Dermatol. 123:1037-1044; Mannon et al., 2004, N. Engl. J Med. 351:2069- 2079; Matusevicius et al., 1999, Mult Scler 5, 101-104; Linden et al., Eur Respir J. 2000 May;15(5):973-7; Molet et al., 2001, J. Allergy Clin. Immunol. 108:430-438). Recent studies have suggested that IL-17F plays a role in the induction of inflammatory responses (Oda et al., 2006, American J. Resp. Crit. Care Medicine, Jan. 15, 2006; Numasaki et al., 2004, Immunol Lett. 95:97-104). WO 2011/088120 describes antibody formulation and therapeutic regimens. Leonardi et al. (2012) NEJM 366(13):1190-1199 describes anti-interleukin-17 monoclonal antibody ixekizumab in chronic plaque psoriasis. NCT02052609 (31st January 2004) describes a Phase 3 study of KHK 4827. Papp 2012 *et al.* (2012) describes brodalumab, an anti-interleukin-17-receptor antibody for psoriasis. Umezawa 2014 *et al.* (12th October 2014) Wireless News describes clinical study data for brodalumab in psoriasis. Gudjonsson et al. (2015) J Invest Dermatol. 118(2):362-5 reports that HLA-Cw6-positive and HLA-Cw6-negative patients with psoriasis vulgaris have distinct clinical features.

There is currently a long-standing unmet need for safe and effective treatments for scalp and nail psoriasis. The invention contemplates treatments of scalp and nail psoriasis using IL-17 Receptor A (IL-17RA or IL-17R) antigen binding proteins, such as the monoclonal antibody brodalumab, as well as other agents that inhibit the IL-17 signaling axis.

## SUMMARY OF INVENTION

The invention provides a composition for use in the treatment of nail or scalp psoriasis in a patient suffering from psoriasis, wherein the composition comprises an antibody or fragment thereof that specifically binds to IL-17 Receptor A (IL-17RA) and has an antagonistic activity,
wherein the antibody comprises a light chain CDR1 comprising the amino acid sequence of SEQ ID NO:1, a light chain CDR2 comprising the amino acid sequence of SEQ ID NO: 2, a light chain CDR3 comprising the amino acid sequence of SEQ ID NO: 3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6,
and wherein the patient has a NAPSI score of at least 6 on one or more nails and/or a PSSI score of at least 15.

Herein disclosed are methods of treating nail or scalp psoriasis or therapeutic agents for nail or scalp psoriasis comprising administering an IL-17RA antigen binding protein, such as a monoclonal antibody or fragment thereof that specifically binds to the IL-17RA. Herein disclosed are methods of treating nail or scalp psoriasis or therapeutic agents for nail or scalp psoriasis comprising administering monoclonal antibodies or fragment thereof that specifically bind to the IL-17RA and have antagonistic activities. Such antagonistic activities include inhibiting binding of IL-17A to the IL-17RA. An exemplary monoclonal antibody, known as AM-14 or brodalumab, comprises a light chain variable domain comprising the amino acid sequence of SEQ ID NO:7 and a heavy chain variable domain comprising the amino acid sequence of SEQ IDN0:8. The full length light chain of brodalumab is set out as the amino acid sequence of SEQ ID NO: 9 and the full length heavy chain of brodalumab is set out as the amino acid sequence of SEQ ID NO: 10. The study described herein demonstrates that brodalumab is more efficacious than a placebo in treating scalp or nail psoriasis after 12 weeks of treatment.

An antibody or fragment thereof may be used for the preparation of a medicament for the treatment of nail or scalp psoriasis, wherein the antibody or fragment thereof the specifically binds to IL-17RA and has an antagonistic activity. Herein disclosed is a composition for use in the treatment of nail or scalp psoriasis, wherein the composition comprises an antibody or fragment thereof that specifically binds to IL-17RA and has an antagonistic activity. Such antagonistic activity includes inhibiting binding of IL-17A to IL-17RA.

Herein disclosed are methods of treating nail or scalp psoriasis, or therapeutic agents for nail or scalp psoriasis, comprising administering to a patient having nail or scalp psoriasis a composition comprising an antibody or fragment thereof selected from: an antibody, comprising a light chain variable domain comprising the amino acid sequence of SEQ IDN0:7 and a heavy chain variable domain comprising the amino acid sequence of SEQ IDNO: 8; an antibody comprising the full length light chain of SEQ ID NO: 9 and a full length heavy chain of SEQ ID NO: 10 and an antibody, comprising a light chain CDR1 comprising the amino acid sequence of SEQ IDN0:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ IDN0:6, wherein said antibody specifically binds to human IL-17RA. For example in the methods of treating nail or scalp psoriasis or therapeutic agents for nail and scalp psoriasis, the monoclonal antibody is a human monoclonal antibody that specifically binds to human IL-17RA and inhibits the binding of IL-17A to said IL-17RA.

An antibody or fragment thereof may be used for the preparation of a medicament for the treatment of nail or scalp psoriasis, wherein the composition comprises an antibody or fragment thereof selected from: an antibody, comprising a light chain variable domain comprising the amino acid sequence of SEQ ID NO:7 and a heavy chain variable domain comprising the amino acid sequence of SEQ ID NO: 8; an antibody comprising the full length light chain of SEQ ID NO: 9 and a full length heavy chain of SEQ ID NO: 10 and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said antibody specifically binds to human IL-17RA. For example in the uses of treating nail or scalp psoriasis, the antibody is a human monoclonal antibody that specifically binds to human IL-17RA and inhibits the binding of IL-17 A to said IL-17RA.

In another aspect of the invention, the invention provide for compositions for use in the treatment or nail or scalp psoriasis comprising an antibody or fragment thereof, wherein the composition comprises an antibody or fragment thereof selected from: an antibody, comprising a light chain variable domain comprising the amino acid sequence of SEQ ID NO:7 and a heavy chain variable domain comprising the amino acid sequence of SEQ ID NO: 8; an antibody comprising the full length light chain of SEQ ID NO: 9 and a full length heavy chain of SEQ ID NO: 10 and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said antibody specifically binds to human IL-17RA. For example, in the composition for treating nail or scalp psoriasis, the antibody is a human monoclonal antibody that specifically binds to human IL-17RA and inhibits the binding of IL-17 A to said IL-17RA.

Nail psoriasis in the present invention means psoriasis developing on the nail in psoriasis patients and includes one or more occurrence of pitting, leukonychia, red spots in lunula, nail plate crumbling, oil drop (salmon patch) discoloration, onycholysis, opacity, thickening, nail bed hyperkeratosis, and splinter hemorrhages. In addition, scalp psoriasis means psoriasis developing on the scalp in psoriasis patients. The kinds of psoriasis of the nail and scalp include psoriasis vulgaris, psoriasis arthropica, pustular psoriasis, psoriatic erythroderma, guttate psoriasis, palmoplantar psoriasis, and plaque psoriasis and the like. A patient having nail psoriasis or scalp psoriasis in the present invention may also have developed psoriasis on other areas of their body; for example, the patient may have psoriasis vulgaris, psoriasis arthropica, pustular psoriasis, psoriatic erythroderma, guttate psoriasis and plaque psoriasis and the like on an area of the body in addition to having one or more of these types of psoriasis on the nails or scalp. Alternatively, the patient may have psoriasis primarily or only on the nail or scalp.

In the methods, uses, compositions or therapeutic agents of the disclosure, the severity of nail psoriasis is assessed using the Nail Psoriasis Severity Index (NAPSI) score or the modified NAPSI (mNAPSI). The NAPSI scale, first proposed by Rich and Scher (J. AM. Acad. Dermalol. 49(2): 206-12, 2003), is determined by first dividing the nail with imaginary horizontal and vertical lines into 4 quarters. The following eight clinical features of nail psoriasis are then scored based on the number of quarters in which the feature is present (0 to 4) to arrive at a NAPSI score of Oto 32 for each nail: pitting, leukonychia, red spots in lunula, nail plate crumbling, oil drop (salmon patch) discoloration, onycholysis, nail bed hyperkeratosis, and splinter hemorrhages. Onycholysis with a red border is more specific for nail psoriasis than onycholysos without erythema. The severity of nail psoriasis is assessed using the modified
NAPSI (mNAPSI) scale, which takes into account the degree of gradation of each parameter from Oto 3 (0 = none, 1 = mild, 2 = moderate and 3 = severe). For example, patients with a NAPSI score of at least 6 or mNAPSI score of at least 2 or 3 for at least one affected nail were considered to have moderate to severe nail psoriasis.

In the methods, uses, compositions or therapeutic agents of the disclosure, the severity of scalp psoriasis is assessed using the Psoriasis Scalp Severity Index (PSSI) score and/or the affected Scalp Surface Area (SSA) score. The PSSI is a scalp-specific modification of the Psoriasis Area Severity Index (PASI), based on the extent of involvement and the severity of erthyema, induration and desquamation. The SSA numerical score (0% to 100%) measures the assessment of the proportion of the subject's total SSA involved with psoriasis. Patients with a PSSI 2: 15 and an SSA 2: 30% were considered to have moderate to severe scalp psoriasis.

The methods for treating nail or scalp psoriasis are treatment methods capable of reducing the degree of severity of psoriasis, which is developed on the nail or the scalp. In addition, the therapeutic agents for nail or scalp psoriasis in the present invention are therapeutic agents capable of reducing the degree of severity of psoriasis which developed on the nail or the scalp. In addition, the treatment methods are also treatment methods capable of improving symptoms of psoriasis developed on the nail or scalp. In addition, the therapeutic agents of the present invention are therapeutic agents capable of improving symptoms of psoriasis developed on the nail or scalp.

An improvement in symptoms of psoriasis developed on the nail (nail psoriasis) refers to a decrease in numerical value of the NAPSI score or mNAPSI score after the administration of an IL-17RA antigen binding protein of the invention relative to before the administration, and preferably the numerical value of the score is decreased in a group to which the IL-17RA antigen binding protein in the invention is administered in comparison to a placebo administered group. In addition, the improvement refers to a decrease in the mean percent change in the NAPSI score or mNAPSI score from baseline, and preferably the mean percent change in the NAPSI score or mNAPSI score from baseline is decreased in the group to which the IL-17RA antigen binding protein is administered in comparison to the placebo administered group.

In addition, the improvement refers to an increase in the NAPSI or mNAPSI score mean percent improvement from the initial administration (baseline), and preferably the NAPSI or mNAPSI mean percent improvement from baseline is increased in the group to which the IL-17RA antigen binding protein is administered in comparison to the placebo administered group.

An improvement in symptoms of psoriasis developed on the scalp (scalp psoriasis) refers to a decrease in numerical value of the PSSI score after the administration of the IL-17RA antigen binding protein relative to before the administration, and preferably the numerical value of the score is decreased in a group to which the IL-17RA antigen binding protein is administered in comparison to a placebo administered group (baseline). In addition, the improvement refers to a decrease in the mean percent change in the PSSI score from baseline, and preferably the mean percent change in the PSSI score from baseline is decreased in the group to which the IL-17RA antigen binding protein is administered in comparison to the placebo administered group.

In addition, the improvement refers to an increase in the PSSI mean percent improvement from the initial administration (baseline), and preferably the PSSI mean percent improvement from baseline is increased in the group to which the IL-17RA antigen binding protein is administered in comparison to the placebo administered group.

The mean percent change in the NAPSI or PSSI score from baseline is calculated as follows:

**Formula 1:**

The mean percent change in the NAPSI, mNAPSI or PSSI score from baseline (%)=(the NAPSI, mNAPSI or PSSI score after administration of the IL-17RA antigen binding protein /the NAPSI, mNAPSI or PSSI score on baseline -1) x 100.

The NAPSI, mNAPSI or PSSI percent improvement from baseline is calculated as follows:

**Formula 2:**

The NAPSI, mNAPSI or PSSI percent improvement from baseline (%) =(1 - the NAPSI, mNAPSI or PSSI score after administration of the IL-17RA antigen binding protein /the NAPSI, mNAPSI or PSSI score on baseline) x100.

For example, the baseline is the initial administration day.

In addition, the improvement refers to an increase in PSSI-75 or PSSI-100, and preferably the PSSI-75 or PSSI-100 is increased in the group to which the IL-17RA antigen binding protein is administered in comparison to the placebo administered group. PSSI-75 or PSSI-100 means percent of patients who achieve 75% or 100% of the PSSI mean percent improvement, respectively.

The placebo administered group may be any drug as long as a drug does not contain an active ingredient; however, specific examples include a solvent of an antibody formulation and the like. The administration may be single administration or multiple administrations (hereinafter, described as "continuous administration").

The therapeutic agent of the present invention includes a therapeutic agent in which the mean percent change in NAPSI or mNAPSI from baseline is reduced by 20% or more, 30 % or more, 40% or more, 50% or more, 60% or more, 70% or more, 80% or more, 90% or more or 100%. Further, the therapeutic agent may include a therapeutic agent in which the mean percent change in the NAPSI or mNAPSI from baseline is reduced by 30% or more, 40% or more, 50% or more, 60% or more, 70% or more, 80% or more, 90% or more or 100% in the group to which the therapeutic agent is administered, with respect to the placebo administration group.

The therapeutic agent of the present invention includes a therapeutic agent in which the mean percent change in PSSI from baseline is reduced by 30% or more, 40% or more, 50% or more, 60% or more, 70% or more, 80% or more, 90% or more or 100%. In addition, the therapeutic agent may include a therapeutic agent in which the mean percent change in the PSSI from baseline is reduced by 20% or more, 30% or more, 40% or more, 50% or more, 60% or more, 70% or more, 80% or more, 90% or more or 100% in the group to which the therapeutic agent is administered, with respect to the placebo administered group.

The treatment method of the present invention includes a treatment method in which the mean percent change in NAPSI or mNAPSI from baseline is reduced by 20% or more, 30 % or more, 40% or more, 50% or more, 60% or more, 70% or more, 80% or more, 90% or more or 100% in the group to which the antibody or the antibody fragment thereof is administered. In addition, the treatment method may include a treatment method in which the mean percent change in the NAPSI or mNAPSI is from baseline reduced by 30% or more, 40% or more, 50% or more, 60% or more, 70% or more, 80% or more, 90% or more or 100% in the group to which the antibody or the antibody fragment thereof is administered, with respect to the placebo administered group.

The treatment method includes a treatment method in which the mean percent change in PSSI from baseline after the administration is reduced by 30 % or more, 40% or more, 50% or more, 60% or more, 70 % or more, 80% or more, 90% or more or 100% in the group to which the antibody or the antibody fragment thereof is administered. In addition, the treatment method may include a treatment method in which the mean percent change in the PSSI from baseline is reduced by 20% or more, 30% or more, 40% or more, 50% or more, 60% or more, 70% or more, 80% or more, 90% or more or 100% in the group to which the antibody or the antibody fragment thereof is administered, with respect to the placebo administered group.

Any of the therapeutic agents, medicaments or compositions of the invention may be administered to induce a mean percent change in NAPSI or mNAPSI from baseline is reduced by 20% or more, 30 % or more, 40% or more, 50% or more, 60% or more, 70% or more, 80% or more, 90% or more or 100% in the group to which the antibody or the antibody fragment thereof is administered. In addition, any of the therapeutic agents, medicaments or compositions of the invention may be administered to induce a mean percent change in the NAPSI or mNAPSI is from baseline reduced by 30% or more, 40% or more, 50% or more, 60% or more, 70% or more, 80% or more, 90% or more or 100% in the group to which the antibody or the antibody fragment thereof is administered, with respect to the placebo administered group.

Any of the therapeutic agents, medicaments or compositions of the invention may be administered to induce a mean percent change in PSSI from baseline after the administration is reduced by 30 % or more, 40% or more, 50% or more, 60% or more, 70 % or more, 80% or more, 90% or more or 100% in the group to which the antibody or the antibody fragment thereof is administered. In addition, any of the therapeutic agents, medicaments or compositions of the invention may be administered to induce a mean percent change in the PSSI from baseline is reduced by 20% or more, 30% or more, 40% or more, 50% or more, 60% or more, 70% or more, 80% or more, 90% or more or 100% in the group to which the antibody or the antibody fragment thereof is administered, with respect to the placebo administered group.

In one embodiment, the patient treated with the preceding method or the patient administered a therapeutic agent, medicament or composition of the invention has 10% or greater body surface area affected by moderate to severe plaque psoriasis prior to treatment. Moderate to severe nail psoriasis includes patients with a NAPSI score of at least 6 or mNAPSI score of at least 2 or 3 on one or more nails. For example, the patient with moderate to severe psoriasis has the presence of at least two of the following clinical features in at least one quarter of a nail: pitting, leukonychia, red spots in lunula, nail plate crumbling, oil drop (salmon patch) discoloration, onycholysis, nail bed hyperkeratosis, splinter hemorrhages or has at least one of these clinical features in more than one quarter of the one or more nails. Moderate to severe scalp psoriasis include patients having a PSSI score of at least 15 and/or a SSA score of at least 30%.

Also disclosed herein are methods of treating nail psoriasis, comprising administering to a patient having a pretreatment NAPSI score of at least 6 or pretreatment mNAPSI score of at least 2 or 3 for at least one affected nail, a composition comprising an human antibody or fragment thereof selected from: an antibody, comprising a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10 and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said human monoclonal antibody specifically binds to human IL-17 receptor A and inhibits the binding of IL-17A to said IL-17 receptor A, wherein the compositions is administered at a dose effective to reduce and maintain a NAPSI score 6 or less or mNAPSI of 3 or less on an affected nail.

In any of the preceding methods, the patient has about 20% or greater body surface area affected by psoriasis, such as plaque psoriasis, or the patient has about 30% or greater body surface area affected by psoriasis or the patient has about 40% or greater body surface area affected by psoriasis, or the patient has about 50% or greater body surface area affected by psoriasis, or the patient has about 60% or greater body surface area affected by psoriasis, or the patient has about 70% or less body surface area affected by psoriasis or the patient has about 80% or greater body surface area affected by psoriasis, or the patient has about 90% or greater body surface area affected by psoriasis.

In another aspect, the invention provides for a therapeutic agent, medicament and composition for treating nail psoriasis in a patient having a pretreatment NAPSI score of at least 6 or pretreatment mNAPSI scores of at least 2 or 3 for at least one affected nail, a composition comprising an antibody or fragment thereof selected from: an antibody, comprising a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10 and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said human monoclonal antibody specifically binds to human IL-17RA, e.g. the monoclonal antibody is a human monoclonal antibody that specifically binds to human IL-17RA and inhibits the binding of IL-17A to said IL-17RA, wherein the composition is administered at a dose effective to reduce and maintain a NAPSI score 6 or less or mNAPSI of 3 or less on an affected nail.

Any of the preceding therapeutic agents, medicaments or compositions are used to treat a patient having about 10% to about 20% body surface area affected by psoriasis, such as plaque psoriasis, or about 20% to about 30% body surface area affected by psoriasis or about 30% to about 40% body surface area affected by psoriasis or about 40% to about 50% body surface area affected by psoriasis or about 50% to about 60% body surface area affected by psoriasis or about 60% to about 70% body surface area affected by psoriasis or about 70% to about 80% body surface area affected by psoriasis or about 80% to about 90% body sulface area affected by psoriasis or about 90% to about 100% body surface area affected by psoriasis.

Also disclosed herein are methods of treating nail or scalp psoriasis, comprising administering to a patient having less than 50% body surface area affected by psoriasis, such as plaque psoriasis, a composition comprising a antibody or fragment thereof selected from: an antibody, comprising a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10 and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said antibody specifically binds to human IL-17RA and inhibits the binding of IL-17 A to said IL-17 receptor A. For example, the monoclonal antibody is a human monoclonal antibody that specifically binds to human IL-17RA and inhibits the binding of IL-17 A to said IL-17RA. For example, in this method the patient has less than 50% body surface area affected by psoriasis, such as plaque psoriasis, or less than 40% body surface area affected by psoriasis or less than 30% body surface area affected by psoriasis, or less than 20% body surface area affected by psoriasis, or less than 10% body surface area affected by psoriasis.

An antibody or fragment thereof may be used for the preparation of a medicament for the treatment of nail or scalp psoriasis, wherein the medicament is for administration to a patient having less than 50% body surface area affected by psoriasis, such as plaque psoriasis, wherein the antibody comprises a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10 and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said antibody specifically binds to human IL-17 receptor A and inhibits the binding of IL-17 A to said IL-17RA. Such antibody may be a human monoclonal antibody that inhibits IL-17 A binding to IL-17RA. For example, this medicament may be administered to patient having less than 50% body surface area affected by psoriasis, such as plaque psoriasis, or less than 40% body surface area affected by psoriasis or less than 30% body surface area affected by psoriasis, or less than 20% body surface area affected by psoriasis, or less than 10% body surface area affected by psoriasis or less than 7% body surface area affected by psoriasis or less than 5% body surface area affected by psoriasis or less than 2% body surface area affected by psoriasis.

Herein disclosed are methods of treating nail or scalp psoriasis, comprising administering to a patient having less than 10% body surface area affected by psoriasis, such as plaques psoriasis, a composition comprising a human antibody or fragment thereof selected from: an antibody, comprising a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10 and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said antibody specifically binds to human IL-17 receptor A. Such antibody may be a human monoclonal antibody that inhibits IL-17 A binding to IL-17RA.

In one embodiment, the patient treated with the preceding method of the invention, or treated with a therapeutic agent, medicament or composition of the invention has 10% or less body surface area affected by moderate to severe nail or scalp psoriasis. Moderate to severe nail psoriasis includes patients with a NAPSI score of at least 6, or a mNAPSI score of at least 3 or at least 2 on one or more nails. For example, the patient with moderate to severe psoriasis has the presence of at least two of the following clinical features in at least one quarter of a nail: pitting, leukonychia, red spots in lunula, nail plate crumbling, oil drop (salmon patch) discoloration, onycholysis, nail bed hyperkeratosis, splinter hemorrhages or has at least one of these clinical features in more than one quarter of the one or more nails. Moderate to severe scalp psoriasis include patients having a **PSSI** score of at least 15 and/or a SSA score of at least 30%.

Herein disclosed are methods of treating nail psoriasis, comprising administering to a patient having a pretreatment NAPSI score of at least 6 or pretreatment mNAPSI score of at least 3 or at least 2 for at least one affected nail, a composition comprising an antibody or fragment thereof selected from: an antibody, comprising a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10 and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said human monoclonal antibody specifically binds to human IL-17RAand inhibits the binding of IL-17 A to said IL-17RA, e.g. the monoclonal antibody is a human monoclonal antibody that specifically binds to human IL-17RA and inhibits the binding of IL-17 A to said IL-17RA, and wherein the composition is administered at a dose and frequency effective to reduce the severity of the patient's nail or scalp psoriasis. In certain embodiments, the patient's score NAPSI score is reduced to 6 or lower. In another embodiment, the patient's mNAPSI score is reduced to 3 or lower on an affected nail. In another embodiment, the patient's NAPSI score is maintained at 6 or lower, or the patient's mNAPSI score is maintained at 3 or lower in an affected nail.

An antibody or fragment thereof may be used for the preparation of a medicament for the treatment of nail psoriasis, wherein the medicament is for administration to a patient having a pretreatment NAPSI score of at least 6 or pretreatment mNAPSI score of at least 3 or at least 2 for at least one affected nail, the medicament comprising an antibody or fragment thereof selected from: an antibody, comprising a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10 and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ IDN0:5, and a heavy chain CDR3 comprising the amino acid sequence of SEQ ID NO:6, wherein said h antibody specifically binds to human IL-17 receptor A, e.g. the monoclonal antibody is a human monoclonal antibody that specifically binds to human IL-17RA and inhibits the binding of IL-17A to said IL-17RA, and wherein the medicament is administered at a dose and frequency effective to reduce the severity of the patient's nail or scalp psoriasis. In certain embodiments, the patient's score NAPSI score is reduced to 6 or lower. In other embodiment, the patient's mNAPSI score is reduced to 3 or lower on an affected nail. In another embodiment, the patient's NAPSI score is maintained at 6 or lower, or the patient's mNAPSI score is maintained at 3 or lower on an affected nail.

Another aspect of the invention provides a composition for treatment of nail psoriasis, wherein the composition is for administration to a patient having a pretreatment NAPSI score of at least 6 or mNAPSI scores of at 3 or at least 2 for at least one affected nail, the composition comprising an human antibody or fragment thereof selected from: an antibody, comprising a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10 and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ IDN0:6, wherein said human monoclonal antibody specifically binds to human IL-17 receptor A and inhibits the binding of IL-17A to said IL-17 receptor A, and wherein the composition is administered at a dose and frequency effective to reduce the severity of the patient's nail or scalp psoriasis. In certain embodiments, the patient's score NAPSI score is reduced to 6 or lower. In other embodiment, the patient's mNAPSI score is reduced to 3 or lower on an affected nail. In other embodiments, the patient's NAPSI score is maintained at 6 or lower, or the patient's mNAPSI score is maintained at 3 or lower.

In any of the preceding methods or any of the therapeutic agents, medicaments or compositions are administered to a patient suffering from psoriasis, such as plaque psoriasis and has about 9.75% or less body surface area affected by psoriasis or the patient has about 9.5% or less body surface area affected by psoriasis or the patient has about 9.25% or less body surface area affected by psoriasis, or the patient has about 9% or less body surface area affected by psoriasis, or the patient has about 8.5% or less body surface area affected by psoriasis, or the patient has about 8% or less body surface area affected by psoriasis or the patient has about 7.5% or less body surface area affected by psoriasis, or the patient has about 7% or less body surface area affected by psoriasis, or the patient has about 6.5% or less body surface area affected by psoriasis or the patient has about 6% or less body surface area affected by psoriasis, or the patient has about 5.5% or less body surface area affected by psoriasis, or the patient about 5% or less body surface area affected by psoriasis, or the patient has about 4.5% or less body surface area affected by psoriasis or the patient has about 4% or less body surface area affected by psoriasis or the patient has about 3.5% or less body surface area affected by psoriasis, or the patient has about 3% or less body surface area affected by psoriasis, or the patient has less than 2.75% body surface area affected by psoriasis, or the patient has about 2.5% or less body surface area affected by psoriasis, or the patient has about 2.5% or less body surface area affected by psoriasis, or the patient has about 2% or less body surface area affected by psoriasis, or the patient has about 1.75% or less body surface area affected by psoriasis, or the patient has about 1.5% or less body surface area affected by psoriasis, or the patient has about 1.25% or less body surface area affected by psoriasis, or the patient has about 1% or less body surface area affected by psoriasis, or the patient has about 0.9% body or less surface area affected by psoriasis, or the patient has about 0.8% or less body surface area affected by psoriasis, or the patient has about 0.7% or less body surface area affected by psoriasis, or the patient has about 0.6% or less body surface area affected by psoriasis, or the patient has about 0.5%% or less body surface area affected by psoriasis, or the patient has about 0.4% or less body surface area affected by psoriasis, or the patient has about 0.3% or less body surface area affected by psoriasis, or the patient has about 0.2% or less body surface area affected by psoriasis, or the patient has about 0.1% or less body surface area affected by psoriasis.

In any of the preceding methods or any of the therapeutic agents, medicaments or compositions are administered to a patient suffering from psoriasis, such as plaque psoriasis and has about 5% to about 9.9% body surface area affected by psoriasis or about 2.5% to about 9% body surface area affected by psoriasis or about 19% to about 9% body surface area affected by psoriasis or about 4% to about 8% body surface area affected by psoriasis or about 2% to about 8% body surface area affected by psoriasis or about 1% to about 8% body surface area affected by psoriasis or about 3% to about 7% body surface area affected by psoriasis or about 2% to about 7% body surface area affected by psoriasis or about 1% to about 7% body surface area affected by psoriasis or about 2% to about 6% body surface area affected by psoriasis or about 1% to about 6% body surface area affected by psoriasis or about 0.5% to about 6% body surface area affected by psoriasis or about 1% to about 5% body surface area affected by psoriasis or about 0.75% to about 5% body surface area affected by psoriasis or about 0.5% to about 5% body surface area affected by psoriasis or about 1% to about 4% body surface area affected by psoriasis or 0.75% to about 4% body surface area affected by psoriasis or about 0.5% to about 4% body surface area affected by psoriasis or about 0.25% to about 4% body surface area affected by psoriasis or 0.5% to about 2% body surface area affected by psoriasis or about 0.25% to about 2% body surface area affected by psoriasis or about 0.5% to about 1% body surface area affected by psoriasis.

Herein disclosed are methods of treating scalp psoriasis, comprising administering to a patient having a pre-treatment PSSI score of at least 15 or least 10 or at least 5 or a pretreatment SSA score of at least 30%, or at least 20% or at least 10%, a composition comprising an antibody or fragment thereof selected from: an antibody, comprising a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10, and an antibody, comprising a light chain CDR I comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said antibody specifically binds to human IL-17RA. The antibody or fragment thereof may be a human monoclonal antibody that and inhibits the binding of IL-17A to said IL-17 receptor A. In certain embodiments, the patient’s PSSI score is reduced to 14 or lower. In another embodiment, the patient’s PSSI score is maintained at 14 or lower. In another embodiment, the patients’ SSA is reduced to 25% or less. In another embodiment, the patient’s SSA is maintained at 25% or less.

An antibody or fragment thereof may be used for the preparation of a medicament for the treating scalp psoriasis, wherein the medicament is for administration to a patient having a pretreatment PSSI score of at least 15 or least 10 or at least 5 or a pretreatment SSA score of at least 30%, or at least 20% or at least 10%, and the antibody or fragment thereof is selected from: an antibody, comprising a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10, and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said antibody specifically binds to human IL-17RA. The antibody or fragment thereof may be a human monoclonal antibody that and inhibits the binding of IL-17 A to said IL-17 receptor A. In certain embodiments, the patient’s PSSI score is reduced to 14 or lower. In another embodiment, the patient’s PSSI score is maintained at 14 or lower. In another embodiment, the patients’ SSA is reduced to 25% or less. In another embodiment, the patient’s SSA is maintained at 2 5% or less.

Another aspect of the invention provides compositions for use in treatment of scalp psoriasis, wherein the composition is for administration to a patient having a pretreatment PSSI score of at least 15 or least 10 or at least 5 or a pretreatment SSA score of at least 30%a, or at least 20% or at least 10%, and the antibody or fragment thereof is selected from: an antibody, comprising a light chain variable domain comprising the amino acid sequence of SEQ ID NO:7 and a heavy chain variable domain comprising the amino acid sequence of SEQ ID NO:8; an antibody comprising the full length light chain of SEQ ID NO: 9 and a full length heavy chain of SEQ ID NO: 10, and an antibody, comprising a light chain CDR1 comprising the amino acid sequence of SEQ ID NO:1, a light chain CDR2 comprising the amino acid sequence of SEQ ID NO:2, a light chain CDR3 comprising the amino acid sequence of SEQ ID NO:3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6, wherein said antibody specifically binds to human IL-17RA. The antibody or fragment thereof may be a human monoclonal antibody that and inhibits the binding of IL-17 A to said IL-17 receptor A. In certain embodiments, the patient's PSSI score is reduced to 14 or lower. In another embodiment, the patient's PSSI score is maintained at 14 or lower. In another embodiment, the patients' SSA is reduced to 2 5% or less.

For example, the preceding methods comprise administering a dose or any of the therapeutic agents, medicaments or compositions comprise a dose of the antibody or fragment thereof that is effective to reduce and maintain a PSSI score of 14 or less, or a PSSI score of 13 or less, a PSSI score of 12 or less, a PSSI score of 10 or less, a PSSI score of 9 or less, a PSSI score of 8 or less, a PSSI score of 7 or less, a PSSI score of 6 or less, a PSSI score of 5 or less, a PSSI score of 4 or less, a PSSI score of 3 or less, a PSSI score of 2 or less, a PSSI score of 1 or less. The dose of the antibody or fragment thereof of any of the preceding methods results in a PSSI score ranging from 12 to 14, or a PSSI score ranging from 10 to 14, or a PSSI score ranging from 8 to 14, or a PSSI score ranging from 6 to 14, or a PSSI score ranging from 4 to 14, or a PSSI score ranging from 2 to 14, or a PSSI score ranging from 10 to 12, or a PSSI score ranging from 8 to 12, or a PSSI score ranging from 6 to 12, or a PSSI score ranging from 4 to 12, or a PSSI score ranging from 2 to 12, or a PSSI score ranging from 8 to 10, or a PSSI score ranging from 6 to 10, or a PSSI score ranging from 4 to 10, or a PSSI score ranging from 2 to 10, or a PSSI score ranging from 6 to 8, or a PSSI score ranging from 4 to 8, or a PSSI score ranging from 2 to 8, or a PSSI score ranging from 4 to 6, or a PSSI score ranging from 2 to 6, or a PSSI score ranging from 2 to 4.

In another embodiment, the preceding methods comprise administering a dose or any of the, therapeutic agents, medicaments or compositions comprise a dose of the antibody or fragment thereof that is effective to reduce or maintain a SSA score of 25% or less, or a SSA score of 20% or less, or a SSA score of 15% or less, or a SSA score of 10% or less, or a SSA score of 5% or less, or a SSA score of 2% or less. The dose of the antibody or fragment thereof of any of the preceding methods results in a SSA score ranging from 20% to 30%, or a SSA score ranging from 15% to 30%, or a SSA score ranging from 10% to 30%, or a SSA score ranging from 20% to 25%, or a SSA score ranging from 15% to 25%, or a SSA score ranging from 10% to 25%, or a SSA score ranging from 5% to 25%, or a SSA score ranging from 15% to 20%, or a SSA score ranging from 10% to 20%, or a SSA score ranging from 5% to 20%, or a SSA score ranging from 12% to 15%, or a SSA score ranging from 10% to 15%, or a SSA score ranging from 5% to 15%, or a SSA score ranging from 2% to 15%, or a SSA score ranging from 7% to 10%, or a SSA score ranging from 5% to 10%, or a SSA score ranging from 2% to 10%, or a SSA score ranging from 5% to 7%, or a SSA score ranging from 2% to 7%, or a SSA score ranging from 2% to 5%.

In any of the preceding methods, the antigen binding protein such as an antibody or fragment thereof is administered to a patient in need thereof by subcutaneous injection such as subcutaneous autoinjection, intralesional injections, topical administration, or systemic administration via intravenous injection or infusion. The antigen binding protein may be administered alone or in combination with another treatment for nail or scalp psoriasis.

Any of the preceding thearpeutic agents, medicaments or composition are administered to a patient in need thereof by subcutaneous injection such as subcutaneous autoinjection, intralesional injections, topical administration, or systemic administration via intravenous injection or infusion. The therapeutic agent, medicament or composition may be administered alone or in combination with another treatment for nail or scalp psoriasis.

In any of the preceding methods, the antibody or fragment thereof is administered with a second treatment. In addition, any of the preceding therapeutic agents, medicaments and compositions may be administered with a second treatment. The second treatment is administered prior to, concurrent with, or subsequent to administration of said composition comprising said antibody or fragment thereof.

In one embodiment, the second treatment is a topical treatment, such as fluorouracil, dithranol, tazarotene, cyclosporine, calcineneurin inhibitors, triamcinolone, fluocinonide, topical steroids, vitamin D3, vitamin D3 analogs, betamethasone dipropionate, betamethasone valerate, calcipotriol, clobetasol, XAMIOL and DAIVOBET, coal tar, urea, corticosteroids, retinoids, anthralin, topical methatrexate, keratolytics, salicylic acid, tofacitinab, apremilast, topical JAK inhibitors and combinations thereof.

In an embodiment, the second treatment is a systemic treatment such as retinoids, acitretin cyclosporine, methotrexate, apremilast, tofacitinib, oral JAK inhibitors, oral PI3 kinase inhibitors, oral MAP kinase inhibitors, Fumaderm, fumarates, dimethyl fumarate, sulfasalazine, leflunomide, calcineurin inhibitors, azathioprine, thioguanine, hydroxyurea, hydroxychloroquine, sulfasalazine and antifungals and combinations thereof. The second treatment is also a biologic such as antagonist, e.g. antibody or chimeric protein, specific for TNF, IL-17, IL-12/23, or IL-23 such as infliximab, adalimumab, etanercept, alefacept, ustekinumab, ixekizumab, secukinumab, guselkumab, and combinations thereof.

In another embodiment, the second treatment is triamcinolone acetonide photochemotherapy, laser therapy, Excimer laser, oral/topical psoralen with UVA (PUVA), pulsed dye laser, radiation therapy, superficial radiotherapy, electron beam therapy, Grenz ray therapy, ermatome shaving, aloe vera extract, narrow band U therapy, UV therapy and combinations thereof.

Any of the second treatments are combined such as a topical therapy combined with one or more systemic treatment, or a topical treatment combined with one or more of triamcinolone acetonide photochemotherapy, laser therapy, Excimer laser, oral/topical psorallen with UVA (PUVA), pulsed dye laser, radiation therapy, superficial radiotherapy, electron beam

therapy, Grenz ray therapy, ermatome shaving, aloe vera extract, UV therapy or a systemic treatment combined with one or more of triamcinolone acetonide photochemotherapy, laser therapy, oral/topical psoralen with UVA (PUVA), pulsed dye laser, radiation therapy, superficial radiotherapy, electron beam therapy, Grenz ray therapy, ermatome shaving, aloe vera extract, narrow band U therapy and UV therapy and combinations thereofs.

In any of the methods, medicaments, compositions or the therapeutic agents, the antibody administered is selected from the group consisting of: a. a human antibody; b. a humanized antibody; c. a chimeric antibody; d. a monoclonal antibody; e. an antigen-binding antibody fragment; f. a single chain antibody; g. a diabody; h. a triabody; i. a tetrabody; j. a Fab fragment; k. a F(ab')2 fragment; 1. an IgD antibody; m. an IgE antibody; n. an IgM antibody; o. an IgG1 antibody; p. an IgG2 antibody; q. an IgG3 antibody; and r.an IgG4

antibody. In any of the methods, the antibody is a human IgG2 monoclonal antibody.

In any of the methods, medicaments, compositions or therapeutic agents, the composition administered is a pharmaceutical composition and that pharmaceutical composition further comprises a pharmaceutically acceptable diluent. In particular, the pharmaceutical composition comprises the following formulation that is an aqueous solution of a glutamic acid buffer and an IL-17RA antigen binding protein at a concentration of 1 00 to 1 50 mg/ml and wherein: a) said glutamic acid buffer comprises a glutamic acid concentration of 5-30 mM.+-.0.2 mM; b) said glutamic acid buffer comprises a pH of 4.5-5.2.+-.0.2; c) said formulation further comprises 2-4% praline (w/v) and 0.005-0.02% (w/v) polysorbate 20. This formulation has an osmolarity of 275 to 325 osm and has a viscosity of 5 to 7 cP at 25°C.

## BRIEF DESCRIPTION OF THE DRAWINGS

Figure 1 provides the overall schema of the clinical study described in Example 1.

Figure 2 provides the PSSI-75 measured in the study subjects every two weeks. The y-axis represents the percent of responders.

Figure 3 provides the PSSI-100 measured in the study subjects every two weeks. The y-axis represents the percent of responders.

Figure 4 provides the PSSI percent improvement from baseline. The y-axis represents the percent of responders.

Figure 5 provides the NAPSI score by treatment group in the induction phase of the study in Example **1.** The y-axis provides the NAPSI score as observed.

Figure 6 provides the NAPSI score for non-rerandomized subjects by treatment group in the induction phase through week 52. The y-axis provides the NAPSI score as observed.

Figure 7 provides the mean percent changes in NAPSI scores from baseline (panel A) and the mean percent changes in PSSI from baseline measured in the phase II study described in Example 2. The horizontal axis shows week from initial administration of Brodalumab or placebo.

## DETAILED DESCRIPTION OF THE INVENTION

The section headings used herein are for organizational purposes only and are not to be construed as limiting the subject matter described.

Standard techniques are used for recombinant DNA, oligonucleotide synthesis, tissue culture and transformation, protein purification, etc. Enzymatic reactions and purification techniques are performed according to the manufacturer's specifications or as commonly accomplished in the art or as described herein. The following procedures and techniques are generally performed according to conventional methods well known in the art and as described in various general and more specific references that are cited and discussed throughout the specification. See, e.g., Sambrook et al., 2001, Molecular Cloning: A Laboratory Manual, 3rd ed., Cold Spring Harbor Laboratory Press, Cold Spring Harbor, N.Y. Unless specific definitions are provided, the nomenclature used in connection with, and the laboratory procedures and techniques of, analytical chemistry, organic chemistry, and medicinal and pharmaceutical chemistry described herein are those well-known and commonly used in the art. Standard techniques are used for chemical synthesis, chemical analyses, pharmaceutical preparation, formulation, and delivery and treatment of patients.

## IL-17A. IL-17F. and IL-17RA

The biologic activities of IL-17A and IL-17F are dependent upon IL-17RA. "IL-17 receptor A" or "IL-17RA" (interchangeably used herein, as well as IL-17 receptor and IL-17R to refer to the same receptor) as used herein is meant the cell surface receptor and receptor complexes (such as but not limited to IL-17RA-IL-17RC complex and IL-17RA-IL-17RB). Without being bound to one particular theory, the different IL-17RA receptor complexes are known to bind one or more of the ligands: IL-17A, IL-17F, IL-17A/F and IL-17 C, and as a result initiate a signal transduction pathway within the cell. IL-17RA proteins also include variants. IL-17RA proteins also include fragments, such as the extracellular domain that don't have all or part of the transmembrane and/or the intracellular domain, as well as fragments of the extracellular domain. The cloning, characterization, and preparation of IL-17RA are described, for example, in U.S. Pat. No. 6,072,033. The amino acid sequence of the human IL-17RA is shown in SEQ ID NO: 13. Soluble forms of huIL-17RA useful in the methods of the present disclosure include the extracellular domain or the mature form lacking the signal peptide or a fragment of the extracellular domain that retains the capacity to bind IL-17A and/or IL-17F, or a heteromeric version of IL-17A and/or IL-17F. Other forms of IL-17RA include muteins and variants that are at least between 70% and 99% homologous to the native IL-17RA of SEQ ID NO:13 and as described in U.S. Pat. No. 6,072,033, so long as the IL-17RA retains the capacity to bind IL-17A and/or IL-17F, or a heteromeric version of IL-17A and/or IL-17F. The term "IL-17RA" also includes post-translational modifications of the IL-17RA amino acid sequence. Post-translational modifications include, but is not limited to, N-and 0-linked glycosylation.

## IL-17RA Antigen Binding Proteins

Herein disclosed are methods of treating scalp and nail psoriasis comprising administering an antigen binding proteins that specifically bind IL-17RA. The methods include administering an IL-17RA antigen binding protein described in U.S. Patent No. 7,767,206.

In a particular aspect, herein disclosed are methods of treating scalp and nail psoriasis comprising administering an antibody comprising a light chain comprising the amino acid sequence of SEQ ID NO: 9 and a heavy chain comprising the amino acid sequence SEQ ID N0:10 or administering an antibody comprising a light chain encoded by the nucleotide sequence of SEQ ID NO: 11 and a heavy chain encoded by the nucleotide sequence of SEQ ID NO: 12. This antibody is described in detail in U.S Patent No. 7,767,206. This antibody is also referred to as brodalumab.

For example, the methods comprise administering an antibody that specifically binds IL-17RA and wherein said antibody comprises a light chain CDR1 having the amino acid sequence of SEQ ID NO: 1, the light chain CDR2 having the amino acid sequence of SEQ ID NO:2, the light chain CDR3 having the amino acid sequence of SEQ ID NO:3 and heavy chain CDR1 having the amino acid sequence of SEQ ID NO:4, the heavy chain CDR2 having the amino acid sequence of SEQ ID NO:5, and the heavy chain CDR3 having the amino acid sequence of SEQ ID NO:6; and fragments, derivatives, muteins, and variants thereof.

For example, embodiments of antigen binding proteins comprise peptides and/or polypeptides (that optionally include post-translational modifications) that specifically bind IL-17RA to a subject in need. Embodiments of antigen binding proteins comprise antibodies and fragments thereof, as variously defined herein, that specifically bind IL-17RA. Antibodies that specifically bind to IL-17RA may have antagonistic activities. The antagonistic activity may be any activity as long as the activity inhibits a biological response of IL-17RA irrespective of whether the binding between IL-17RA and a ligand of the receptor is inhibited or not. Specific examples of the biological response include proliferation, infiltration and migration of an IL-17RA expressing cell, a cytokine production from an IL-17RA expressing cell and the like. Antibodies that specifically bind to human IL-17RA may inhibit IL-17 A and/or IL-17F from binding and activating IL-17RA, or a heteromeric complex of IL-17RA and IL-17RC. Throughout the specification, when reference is made to inhibiting IL-17 A and/or IL-17F, it is understood that this also includes inhibiting heteromers of IL-17 A and IL-17F. Antibodies that specifically bind to human IL-17RA may partially or fully inhibit IL-17RA from forming either a homomeric or heteromeric functional receptor complex, such as, but not limited to, an IL-17RA-IL-17RC complex. Antibodies that specifically bind to human IL-17RA may partially or fully inhibit IL-17RA from forming either a homomeric or heteromeric functional receptor complex, such as, but not limited to IL-17RA/IL-17RC complex and do not necessarily inhibit IL-17 A and/or IL-17F or an IL-17 A/IL-17F heteromer from binding to IL-17RA or a IL-17RA heteromeric receptor complex.

The antigen binding proteins of the invention specifically bind to IL-17RA. "Specifically binds" as used herein means that the antigen binding protein preferentially binds IL-17RA over other proteins. In some embodiments "specifically binds" means that the IL-17RA antigen binding proteins have a higher affinity for IL-17RA than for other proteins. For example, the equilibrium dissociation constant is< 10-7 to 10-11 M, or<10-8 to<10-10 M, or <10-9 to<10-10 M.

It is understood that when reference is made to the various embodiments of the IL-17RA antibodies described herein, that it also encompasses IL-17RA-binding fragments thereof. An IL-17RA-binding fragment comprises any of the antibody fragments or domains described herein that retains the ability to specifically bind to IL-17RA. Said IL-17RA-binding fragments are in any of the scaffolds described herein. Said IL-17RA-binding fragments also have the capacity to inhibit activation of the IL-17RA, as described throughout the specification.

In a further variation, the antigen binding protein comprises A) a heavy chain amino acid sequence that comprises a H-CDR1, a H-CDR2, and a H-CDR3 of any of SEQ ID NO:4-6, and B) a light chain amino acid sequence that comprises a L-CDR1, a L-CDR2, and a L-CDR3 of any of SEQ ID NO:1- 3. In another variation, the antigen binding protein comprises an amino acid sequence that is of at least 80%, 81%, 8 2%, 8 3%, 84%, 85%, 86%, 87%, 88%, 89%, 90%, 91%, 9 2%, 9 3%, 9 4%, 95%, 96%, 97%, 98%, or 99% identical to a heavy chain amino acid sequence selected from the group consisting of SEQ ID NO:4-6 or a light chain amino acid sequence selected from the group consisting of SEQ ID NO:1- 3.

In another embodiment, herein disclosed is an antigen binding protein that specifically binds IL-17RA, wherein said antigen binding protein comprises a light chain CDR1, CDR2, CDR3 and a heavy chain CDR1, CDR2, and CDR3 that differs by no more than a total of one, two, three, four, five, or six amino acid additions, substitutions, and/or deletions from the following CDRsequences:; light chain CDR1 (SEQ ID NO:1), CDR2 (SEQ ID NO:2), CDR3 (SEQ ID NO:3) and heavy chain CDR1 (SEQ ID NO:4), CDR2 (SEQ ID NO:5), CDR3 (SEQ ID NO:5) of antibody AM-14; and fragments, derivatives, muteins, and variants thereof.

In another embodiment, the light chain variable domain comprises a sequence of amino acids that is encoded by a polynucleotide that hybridizes under moderately stringent conditions to a complement of a light chain polynucleotide sequence of SEQ ID NO: 11

In another embodiment, the heavy chain variable domain comprises a sequence of amino acids that is encoded by a polynucleotide that hybridizes under moderately stringent or stringent conditions to a complement of a heavy chain polynucleotide sequence of SEQ ID NO: 12.

Accordingly, in various embodiments, the antigen binding proteins of the invention comprise the scaffolds of traditional antibodies, including human and monoclonal antibodies, bispecific antibodies, diabodies, minibodies, domain antibodies, synthetic antibodies (sometimes referred to herein as "antibody mimetics"), chimeric antibodies, antibody fusions (sometimes referred to as "antibody conjugates"), and fragments of each, respectively. The above described CDRs and combinations of CDRs are grafted into any of the following scaffolds.

As used herein, the term "antibody" refers to the various forms of monomeric or multimeric proteins comprising one or more polypeptide chains that specifically binds to an antigen, as variously described herein. In certain embodiments, antibodies are produced by recombinant DNA techniques. In additional embodiments, antibodies are produced by enzymatic or chemical cleavage of naturally occurring antibodies. In another aspect, the antibody is selected from the group consisting of: a) a human antibody; b) a humanized antibody; c) a chimeric antibody; d) a monoclonal antibody; e) a polyclonal antibody; f) a recombinant antibody; g) an antigen-binding antibody fragment; h) a single chain antibody; i) a diabody; j) a triabody; k) a tetrabody; 1) a Fab fragment; m) a F(ab')2 fragment; n) an IgD antibody; o) an IgE antibody; p) an IgM antibody; q) an IgA antibody; r) an IgGI antibody; s) an IgG2 antibody; t) an IgG3 antibody; and u) an IgG4 antibody.

"Humanized antibodies" generally refer to non-human antibodies that have had the variable-domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies is described in, e.g., WO 92/1101 8, Jones, 1 986, Nature 321 :522-525, Verhoeyen et al., 1 988, Science 239: 1534-1536. Humanized antibodies can also be generated using mice with a genetically engineered immune system. Roque et al., 2004, Biotechnol. Prog. 20:639-654. In the present invention, the identified CDRs are human, and thus both humanized and chimeric antibodies in this context include some non-human CDRs; for example, humanized antibodies are generated that comprise the CDRH3 and CDR13 regions, with one or more of the other CDR regions being of a different special origin.

In one embodiment, the IL-17RA antigen binding protein is a multispecific antibody, and notably a bispecific antibody, also sometimes referred to as "diabodies". These are antibodies that bind to two (or more) different antigens. Diabodies can be manufactured in a variety of ways known in the art (Holliger and Winter, 1993, Current Opinion Biotechnol. 4:446-449), e.g., prepared chemically or from hybrid hybridomas.

In one embodiment, the IL-17RA antigen binding protein is a minibody. Minibodies are minimized antibody-like proteins comprising a scFv joined to a CH3 domain. Hu et al., 1996, Cancer Res. 56:3055-3061.

In one embodiment, the IL-17RA antigen binding protein is an antibody fragment, that is a fragment of any of the antibodies outlined herein that retain binding specificity to IL-17RA. In various embodiments, the antibody binding proteins comprise, but are not limited to, a F(ab), F(ab'), F(ab')2, Fv, or a single chain Fv fragments. At a minimum, an antibody, as meant herein, comprises a polypeptide binds specifically to IL-17RA comprising all or part of a light or heavy chain variable region, such as one or more CDRs.

Further examples of IL-17RA-binding antibody fragments include, but are not limited to, (i) the Fab fragment consisting of VL, VH, CL and CHI domains, (ii) the Fd fragment consisting of the VH and CHI domains, (iii) the Fv fragment consisting of the VL and VH domains of a single antibody; (iv) the dAb fragment (Ward et al., 1989, Nature 341:544-546) which consists of a single variable, (v) isolated CDR regions, (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., 1988, Science 242:423-426, Huston et al., 1988, Proc. Natl. Acad. Sci. U.S.A. 85:5879-5883), (viii) bispecific single chain Fv dimers (PCT/US92/09965) and (ix) "diabodies" or "triabodies", multivalent or multispecific fragments constructed by gene fusion (Tomlinson et. al., 2000, Methods Enzymol. 326:461-479; WO94/13804; Bolliger et al., 1993, Proc. Natl. Acad. Sci. U.S.A. 90:6444-6448). The antibody fragments are modified. For example, the molecules are stabilized by the incorporation of disulphide bridges linking the VH and VL domains (Reiter et al., 1996, Nature Biotech. 14:1239-1245). Aspects of the invention include embodiments wherein the non-CDR components of these fragments are human sequences.

In one embodiment, the IL-17RA antigen binding protein is a fully human antibody. In this embodiment, as outlined above, specific structures comprise complete heavy and light chains depicted comprising the CDR regions. Additional embodiments utilize one or more of the CDRs of the disclosure, with the other CDRs, framework regions, J and D regions, constant regions, etc., coming from other human antibodies. For example, the CDRs of the disclosure can replace the CDRs of any number of human antibodies, particularly commercially relevant antibodies.

Single chain antibodies are formed by linking heavy and light chain variable domain (Fv region) fragments via an amino acid bridge (short peptide linker), resulting in a single polypeptide chain. Such single-chain Fvs (scFvs) have been prepared by fusing DNA encoding a peptide linker between DNAs encoding the two variable domain polypeptides (VL and VH). The resulting polypeptides can fold back on themselves to form antigen-binding monomers, or they can form multimers (e.g., dimers, trimers, or tetramers), depending on the length of a flexible linker between the two variable domains (Kortt et al., 1997, Prot. Eng. 10:423; Kortt et al., 2001, Biomol. Eng. 18:95-108). By combining different VL and VB-comprising polypeptides, one can form multimeric scFvs that bind to different epitopes (Kriangkum et al., 2001, Biomol. Eng. 18:31-40). Techniques developed for the production of single chain antibodies include those described in U.S. Patent No. 4,946,778; Bird, 1988, Science 242:423; Huston et al., 1988, Proc. Natl. Acad. Sci. USA 85:5879; Ward et al., 1989, Nature 334:544, de Graaf et al., 2002, Methods Mol Biol. 178:379-87.

By "protein," as used herein, is meant at least two covalently attached amino acids, which includes proteins, polypeptides, oligopeptides and peptides. In some embodiments, the two or more covalently attached amino acids are attached by a peptide bond. The protein is made up of naturally occurring amino acids and peptide bonds, for example when the protein is made recombinantly using expression systems and host cells, as outlined below. Alternatively, the protein includes synthetic amino acids (e.g., homophenylalanine, citrulline, omithine, and norleucine), or peptidomimetic structures, i.e., "peptide or protein analogs", such as peptoids (see, Simon et al., 1992, Proc. Natl. Acad. Sci. U.S.A. 89:9367), which can be resistant to proteases or other physiological and/or storage conditions.
Such synthetic amino acids is incorporated in particular when the antigen binding protein is synthesized in vitro by conventional methods well known in the art. In addition, any combination of peptidomimetic, synthetic and naturally occurring residues/structures can be used. "Amino acid" also includes imino acid residues such as praline and hydroxyproline. The amino acid "R group" or "side chain" is either the (L)- or the (S)-configuration. In a specific embodiment, the amino acids are in the (L)- or (S)-configuration.

In certain aspects, herein disclosed are recombinant antigen binding proteins that bind an IL-17RA, in some embodiments a recombinant human IL-17RA or portion thereof. In this context, a "recombinant protein" is a protein made using recombinant techniques using any techniques and methods known in the art, i.e., through the expression of a recombinant nucleic acid as described herein. Methods and techniques for the production of recombinant proteins are well known in the art. Embodiments of the invention include recombinant antigen binding proteins that bind wild-type IL-17RA and variants thereof.

## Use of IL-17RA Antigen Binding Proteins For Diagnostic And Therapeutic Purposes

IL-17RA antigen binding proteins may be used for the prevention or treatment of diseases or conditions associated with the IL-17A and/or IL-17F activity. For example, the IL-17RA antigen binding domains of the present are used for the prevention and treatment of scalp and/or nail psoriasis.

Antigen binding proteins, e.g. antibodies and fragments thereof, that specifically bind to IL-17RA may be used in treatment of scalp or nail psoriasis in a patient in need thereof. The antigen binding proteins, e.g. antibodies and fragmentes thereof, that specifically bind to IL-17A may be administered systemically via intravenous injection or infusion, subcutaneous injection such as subcutaneous autoinjection, intralesional injections or topical administration. The antigen binding protein may be administered alone or in combination with another treatment for nail or scalp psoriasis.

All aspects of the IL-17RA antigen binding proteins described throughout this specification may be used in the preparation of a medicament for the treatment of scalp or nail psoriasis described herein. In addition, the IL-17RA antigen binding protein are used to inhibit IL-17RA from forming a complex with its ligand, e.g., IL-17A and/or IL-17F or any other IL-17 ligand family member that binds IL-17RA or a heterologous complex comprising IL-17RA and IL-17RC, thereby modulating the biological activity of IL-17RA in a cell or tissue. Antigen binding proteins that bind to IL-17RA thus may modulate and/or inhibit interaction with other binding compounds and as such may have therapeutic use in ameliorating scalp or nail psoriasis. In specific embodiments, IL-17RA antigen binding proteins may inhibit IL-17A and/or IL-17F and/or IL-17A/F from binding IL-17RA, which may result in disruption of the IL-17RA-induced signal transduction cascade.

Topical treatments for nail psoriasis include fluorouracil, dithranol, anthralin, tazarotene, cyclosporine, calcineeneurin inhibitors triamcinolone, fluocinonide, topical steroids, corticosteroids, vitamin D3, vitamin D3 analogs such as betamethasone dipropionate, betamethasone valerate, calcipotriol (e.g DAIVOBET) clobetasol, combination therapies such as XAMIOL (betamethasome dipropionate and calcipotriol gel) and combinations thereof.

Systemic treatments for nail psoriasis include a systemic treatment such as retinoids, acitretin cyclosporine, antifungals, methotrexate and biologic therapies such as such as antagonists, e.g. antibody or chimeric protein, specific for TNF IL-17 IL-12/23 or IL-23 such as infliximab, adalimumab, etanercept, alefacept and ustekinumab and combinations thereof.

Other types of treatment for nail psoriasis include triamcinolone acetonide photochemotherapy, narrow band phototherapy, photochemotherapy with UVA, photosensitizer psoralan with UVA (PUVA), laser therapy, pulsed dye laser, radiation therapy, superficial radiotherapy, electron beam therapy and Grenz ray therapy, and combinations thereof.

Topical treatments for scalp psoriasis include corticosteroids such as hydrocortisone, clobetasone, triamcinolone, betamethasome verate, betamethasone dipropionate, desoximethasone, salicylic acid, coal tar, zinc pyrithion, antifungals, dithranol, antimycotics, vitamin D3, vitamin D3 analogs, urea, retinoids, anthralin, topical methotrexate and keratolytics and combinations thereof.

Systemic treatment for scalp psoriasis include methrotrexate, cyclosporine, acritretin, and biologic therapies such as such as antagonists, e.g. antibody or chimeric protein, specific for TNF IL-17 IL-12/23 or IL-23 such as infliximab, adalimumab, etanercept, and alefacept and combinations thereof.

Other types of treatment for scalp psoriasis include photochemical therapy, photosensitizer psoralan (PUVA) psoralelectron beam therapy and Grenz ray therapy, dermatome shaving, aloe vera extract and UV therapy and combinations thereof.

Treatment of scalp or nail psoriasis includes the use of first line drugs for topical treatments or other drugs for control of pain and inflammation in combination (pretreatment, post-treatment, or concurrent treatment) with treatment with one or more of the antigen binding proteins provided herein. These drugs are classified as non-steroidal, anti-inflammatory drugs (NSAIDs). Secondary treatments include corticosteroids, slow acting antirheumatic drugs (SAARDs), or disease modifying (DM) drugs. Information regarding the following compounds can be found in The Merck Manual of Diagnosis and Therapy, Sixteenth Edition, Merck, Sharp & Dohme Research Laboratories, Merck & Co., Rahway, N.J. (1992) and in Pharmaprojects, PJB Publications Ltd.

The antigen binding proteins described herein are used in combination (pre-treatment, post-treatment, or concurrent treatment) with any of one or more TNF inhibitors for the treatment or prevention of scalp or nail psoriasis, such as but not limited to, all forms of soluble TNF receptors including Etanercept (such as ENBREL®), as well as all forms of monomeric or multimeric p75 and/or p55 TNF receptor molecules and fragments thereof; anti- human TNF antibodies, such as but not limited to, Infliximab (such as REMICADE®), and D2E7 (such as HUMIRA®), and the like. Such TNF inhibitors include compounds and proteins which block in vivo synthesis or extracellular release of TNF. In a specific embodiment, the present invention is directed to the use of an IL-17RA antigen binding protein in combination (pre-treatment, post-treatment, or concurrent treatment) with any of one or more of the following TNF inhibitors: TNF binding proteins (soluble TNF receptor type-I and soluble TNF receptor type-II ("sTNFRs", as defined herein), anti-TNF antibodies, granulocyte colony stimulating factor; thalidomide; BN 50730; tenidap; E 5531; tiapafant PCA 4248; nimesulide; PANAVIR® (Probucol); rolipram; RP 73401; peptide T; MDL 201,449A; (1R,3S)-Cis- I -[9- (2,6-diaminopurinyl)]-3-hydroxy-4-cyclopentene hydrochloride; (1R,3R)-trans-I-(9-(2,6-diamino)purine]-3-acetoxycyclopentane; (1R,3R)-trans-I-[9-adenyl)-3-azidocyclopentane hydrochloride and (1R,3R)-trans- I-(6-hydroxy-purin-9-yl)-3-azidocyclo-pentane. TNF binding proteins are disclosed in the art (EP 308 378, EP 422 339, GB 2 218 101, EP 393 438, WO 90/13575, EP 398 327, EP 412 486, WO 91/03553, EP 418 014, JP 127,800/1991, EP 433 900, U.S. Patent No. 5,136,021, GB 2 246 569, EP 464 533, WO 92/01002, WO 92/13095, WO 92/16221, EP 512 528, EP 526 905, WO 93/07863, EP 568 928, WO 93/21946, WO 93/19777, EP 417 563, WO 94/06476, and WO 98/001555).

For example, EP 393 438 and EP 422 339 teach the amino acid and nucleic acid sequences of a soluble TNF receptor type I (also known as "sTNFR-I" or "30kDa TNF inhibitor") and a soluble TNF receptor type II (also known as "sTNFR-II" or "40kDa TNF inhibitor"), collectively termed "sTNFRs", as well as modified forms thereof (e.g., fragments, functional derivatives and variants). EP 393 438 and EP 422 339 also disclose methods for isolating the genes responsible for coding the inhibitors, cloning the gene in suitable vectors and cell types and expressing the gene to produce the inhibitors. Additionally, polyvalent forms (i.e., molecules comprising more than one active moiety) of sTNFR-I and sTNFR-II have also been disclosed. In one embodiment, the polyvalent form is constructed by chemically coupling at least one TNF inhibitor and another moiety with any clinically acceptable linker, for example polyethylene glycol (WO 92/16221 and WO 95/34326), by a peptide linker (Neve et al. (1996), Cytokine, 8(5):365-370, by chemically coupling to biotin and then binding to avidin (WO 91/03553) and, finally, by combining chimeric antibody molecules (U.S. Patent 5,116,964, WO 89/09622, WO 91/16437 and EP 315062).

The antigen binding proteins described herein are used in combination with all forms of CD28 inhibitors, such as but not limited to, abatacept (for example ORENCIA®).

The antigen binding proteins described herein are used in combination with all forms of IL-6 and/or IL-6 receptor inhibitors, such as but not limited to, tocilizumab (for example ACTEMRA®).

The antigen binding proteins described herein are used in combination with all forms of IL-23 and/or IL-12 such as ustekinumab (STELARA) and guselkumab.

The antigen binding protein described herein are used in combination with other IL-17RA inhibitors, such as secukinumab and ixekizumab.

The antigen binding proteins described herein are used in combination with small molecules that bind and/or inhibit IL-17RA or IL-17 activity or small molecules that bind and/or inhibit the activity of other pro-inflammatory cytokines. Examples of these small molecules include synthetic small molecule macrocycle antagonists of human IL-17A as those described in Livingston et al. "Identification and Characterization of Synthetic Small Molecule Macrocycle Antagonists of Human IL17A" ACR Annual Meeting, November 9-14, 2012.

In a specific embodiment, the present invention is directed to the use of an antigen binding protein and any of one or more NSAIDs for the treatment of the diseases and disorders recited herein. NSAIDs owe their anti-inflammatory action, at least in part, to the inhibition of prostaglandin synthesis (Goodman and Gilman in "The Pharmacological Basis of Therapeutics," MacMillan 7th Edition (1985)). NSAIDs can be characterized into at least nine groups: (1) salicylic acid derivatives; (2) propionic acid derivatives; (3) acetic acid derivatives; (4) fenamic acid derivatives; (5) carboxylic acid derivatives; (6) butyric acid derivatives; (7) oxicams; (8) pyrazoles and (9) pyrazolones.

In another specific embodiment, the present invention is directed to the use of an antigen binding protein in combination (pretreatment, post-treatment, or concurrent treatment) with any of one or more salicylic acid derivatives, prodrug esters or pharmaceutically acceptable salts thereof. Such salicylic acid derivatives, prodrug esters and pharmaceutically acceptable salts thereof comprise: acetaminosalol, aloxiprin, aspirin, benorylate, bromosaligenin, calcium acetylsalicylate, choline magnesium trisalicylate, magnesium salicylate, choline salicylate, diflusinal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide 0-acetic acid, salsalate, sodium salicylate and sulfasalazine. Structurally related salicylic acid derivatives having similar analgesic and anti-inflammatory properties are also intended to be encompassed by this group.

In still another specific embodiment, the present invention is directed to the use of an antigen binding protein in combination (pretreatment, post-treatment or concurrent treatment) with any of one or more corticosteroids, prodrug esters or pharmaceutically acceptable salts thereof for the treatment of the diseases and disorders recited herein, including acute and chronic inflammation such as rheumatic diseases, graft versus host disease and multiple sclerosis. Corticosteroids, prodrug esters and pharmaceutically acceptable salts thereof include hydrocortisone and compounds which are derived from hydrocortisone, such as 21- acetoxypregnenolone, alclomerasone, algestone, amcinonide, beclomethasone, betamethasone, betamethasone valerate, budesonide, chloroprednisone, clobetasol, clobetasol propionate, clobetasone, clobetasone butyrate, clocortolone, cloprednol, corticosterone, cortisone, cortivazol, deflazacon, desonide, desoximerasone, dexamethasone, diflorasone, diflucortolone, difluprednate, enoxolone, fluazacort, flucloronide, flumethasone, flumethasone pivalate, flucinolone acetonide, flunisolide, fluocinonide, fluorocinolone acetonide, fluocortin butyl, fluocortolone, fluocortolone hexanoate, diflucortolone valerate, fluorometholone, fluperolone acetate, fluprednidene acetate, fluprednisolone, flurandenolide, formocortal, halcinonide, halometasone, halopredone acetate, hydro-cortamate, hydrocortisone, hydrocortisone acetate, hydro-cortisone butyrate, hydrocortisone phosphate, hydrocortisone 21-sodium succinate, hydrocortisone tebutate, mazipredone, medrysone, meprednisone, methylprednisolone, mometasone furoate, paramethasone, prednicarbate, prednisolone, prednisolone 21- diedryaminoacetate, prednisolone sodium phosphate, prednisolone sodium succinate, prednisolone sodium 21-m-sulfobenzoate, prednisolone sodium 21-stearoglycolate, prednisolone tebutate, prednisolone 21-trimethylacetate, prednisone, prednival, prednylidene, prednylidene 21-diethylaminoacetate, tixocortol, triamcinolone, triamcinolone acetonide, triamcinolone benetonide and triamcinolone hexacetonide. Structurally related corticosteroids having similar analgesic and anti-inflammatory properties are also intended to be encompassed by this group.

The antigen binding proteins are used to reduce IL-17RA activity, comprising administering an antigen binding protein. The present disclosure is also directed to methods of inhibiting binding and/or signaling of IL-17A and/or IL-17F to IL-17RA comprising providing the antigen binding protein of the disclosure to IL-17RA. In certain embodiments, the antigen binding protein inhibits binding and/or signaling of IL-17A and IL-17F to IL-17RA. In additional embodiments, the antigen binding protein inhibits binding and/or signaling of IL-17A but not IL-17F to IL-17RA. In other embodiments, the antigen binding protein inhibits binding and/or signaling of IL-17F and not IL-17A to IL-17RA. The antigen binding proteins are used in treating the consequences, symptoms, and/or the pathology associated with IL-17RA activity, comprising administering an antigen binding protein. The antigen binding proteins are used to inhibit the production of one or more of an inflammatory cytokine, chemokine, matrix metalloproteinase, or other molecule associated with IL-17RA activation, comprising administering an antigen binding protein. The antigen binding proteins are used in methods of inhibiting production of molecules such as but is not limited to: IL-6, IL-8, CXCLI, CXCL2, GM-CSP, G-CSF, M-CSF, IL-1β, TNFa, RANK-L, LIP, PGE2, IL-12, MMPs (such as but not limited to MMP3 and MMP9), GROα, NO, and/or C-telopeptide and the like, comprising administering an antigen binding protein. The antigen binding proteins inhibit proinflammatory and proautoimmune immune responses and are used to treat diseases associated with activity of the IL-17A and/or IL-17F/IL-17RA pathway.

**Methods of Treatment: Pharmaceutical Formulations, Routes of Administration**

Herein disclosed are pharmaceutical compositions comprising a therapeutically effective amount of one or a plurality of the antigen binding proteins together with a pharmaceutically acceptable diluent, carrier, solubilizer, emulsifier, preservative, and/or adjuvant. In addition, herein disclosed are methods of treating a patient by administering such pharmaceutical composition. The term "patient" includes human and animal subjects.

The methods may comprise administering a composition comprising an IL-17RA antigen binding protein formulation including any formulation described in U.S. Patent No. 7,767,206.

In another embodiment, the IL-17 antigen binding protein formulation used in the methods is one of the formulation described in U.S Patent Publication No. US2013/0022621. For example, a pharmaceutical formulation, comprising an aqueous solution of a glutamic acid buffer and an antibody comprising a heavy chain CDR1 comprising SEQ ID NO:4, a heavy chain CDR2 comprising SEQ ID NO:5, a heavy chain CDR3 comprising SEQ ID NO:6, a light chain CDR1 comprising SEQ ID NO:1, a light chain CDR2 comprising SEQ ID NO:2, and a light chain CDR3 comprising SEQ ID NO:3, wherein said antibody, or fragment thereof, specifically binds human IL-17 receptor A, and wherein: a) said glutamic acid buffer comprises a glutamic acid concentration of 5-3 0 mM.+-.0.2 mM; b) said glutamic acid buffer comprises a pH of 4.5-5.2.+-.0.2; c) said formulation further comprises 2-4% praline (w/v) and 0.005-0.02% (w/v) polysorbate 20; and d) said antibody is at a concentration of 100 to 15 0 mg/ml. This formulation has an osmolarity of 275 to 3 25 osm and has a viscosity of 5 to 7 cP at 25oC.

In one embodiment, the IL-17RA antigen binding protein composition comprises an aqueous solution of a glutamic acid buffer and an antibody or a fragment thereof comprising a heavy chain CDR1 comprising SEQ ID NO:4, a heavy chain CDR2 comprising SEQ IDN0:5, a heavy chain CDR3 comprising SEQ ID NO:6, a light chain CDR1 comprising SEQ IDN0:1, a light chain CDR2 comprising SEQ IDN0:3, and a light chain CDR3 comprising SEQ ID NO:3, wherein said antibody, or fragment thereof, specifically binds human IL-17 receptor A, and wherein: said formulation comprises a glutamic acid concentration of 10 ± 0.2 mM; said formulation has a pH of 4.5-5.2 ± 0.2; said formulation further comprises 3 ± 0.2% praline (w/v) and 0.01 ± 0.002% (w/v) polysorbate 20; said antibody is at a concentration of about 140± 5% mg/ml ; and said formulation has a viscosity of 5 to 7 cP at 25 degrees C. This formulation has an osmolarity of 275 to 3 25 osm.

In one embodiment, the IL-17RA antigen binding domain composition comprise an aqueous solution of a glutamic acid buffer and an antibody or a fragment thereof comprising a heavy chain CDR1 comprising SEQ ID NO:4, a heavy chain CDR2 comprising SEQ IDN0:5, a heavy chain CDR3 comprising SEQ ID NO:6, a light chain CDR1 comprising SEQ IDN0:1, a light chain CDR2 comprising SEQ IDN0:2, and a light chain CDR3 comprising SEQ ID NO:3, wherein said antibody, or fragment thereof, specifically binds human IL-17 receptor A, and wherein: said formulation has a glutamic acid concentration of 10 ± 0.2 mM; said formulation has a pH of 4.8 ± 0.2; said formulation further comprises 3 ± 0.2% praline (w/v) and 0.01 ± 0.002% (w/v) polysorbate 20; said antibody is at a concentration of about 140 ± 5% mg/ml ; and said formulation has a viscosity of 5 to 7 cP at 25 degrees C. This formulation has an osmolarity of 275 to 325 osm.

In another embodiment, the IL-17RA antigen binding protein composition comprises an aqueous solution of a glutamic acid buffer and an antibody or a fragment thereof comprising a heavy chain CDR1 comprising SEQ ID NO:4, a heavy chain CDR2 comprising SEQ ID NO:5, a heavy chain CDR3 comprising SEQ ID NO:6, a light chain CDR1 comprising SEQ ID NO:1, a light chain CDR2 comprising SEQ ID NO:3, and a light chain CDR3 comprising SEQ ID NO:3, wherein said antibody, or fragment thereof, specifically binds human IL-17 receptor A, and wherein: said formulation comprises 30 mM glutamic acid; said formulation has a pH of 4.8± 0. 2; said formulation further comprises 2. 4 ± 0. 2% praline (w/v) and 0.01 ± 0.00 2% (w/v) polysorbate 20; said antibody is at a concentration of about 1 40 ± 5% mg/ml; and said formulation has a viscosity of 5 to 7 cP at 25 degrees C. This formulation has an osmolarity of 275 to 325 osm.

Preferably, acceptable formulation materials are nontoxic to recipients at the dosages and concentrations employed. In specific embodiments, pharmaceutical compositions comprising a therapeutically effective amount of IL-17RA antigen binding proteins are provided.

In certain embodiments, acceptable formulation materials preferably are nontoxic to recipients at the dosages and concentrations employed. In certain embodiments, the pharmaceutical composition contain formulation materials for modifying, maintaining or preserving, for example, the pH, osmolarity, viscosity, clarity, color, isotonicity, odor, sterility, stability, rate of dissolution or release, adsorption or penetration of the composition. In such embodiments, suitable formulation materials include, but are not limited to, amino acids (such as glycine, glutamine, asparagine, arginine or lysine); antimicrobials; antioxidants (such as ascorbic acid, sodium sulfite or sodium hydrogensulfite); buffers (such as borate, bicarbonate, Tris-HCl, citrates, phosphates or other organic acids); bulking agents (such as mannitol or glycine); chelating agents (such as ethylenediamine tetraacetic acid (EDT A)); complexing agents (such as caffeine, polyvinylpyrrolidone, beta-cyclodextrin or hydroxypropyl-beta- cyclodextrin); fillers; monosaccharides; disaccharides; and other carbohydrates (such as glucose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. See, REMINGTON'S PHARMACEUTICAL SCIENCES, 18" Edition, (A.R. Genrmo, ed.), 1990, Mack Publishing Company.

In certain embodiments, the optimal pharmaceutical composition will be determined by one skilled in the art depending upon, for example, the intended route of administration, delivery format and desired dosage. See, for example, REMINGTON'S PHARMACEUTICAL SCIENCES, supra. In certain embodiments, such compositions influence the physical state, stability, rate of in vivo release and rate of in vivo clearance of the antigen binding proteins. In certain embodiments, the primary vehicle or carrier in a pharmaceutical composition is either aqueous or non-aqueous in nature. For example, a suitable vehicle or carrier is water for injection, physiological saline solution or artificial cerebrospinal fluid, possibly supplemented with other materials common in compositions for parenteral administration. Neutral buffered saline or saline mixed with serum albumin are further exemplary vehicles. In specific embodiments, pharmaceutical compositions comprise Tris buffer of about pH 7.0-8.5, or acetate buffer of about pH 4.0-5.5, and further includes sorbitol or a suitable substitute therefor. In certain embodiments of the invention, IL-17RA antigen binding protein compositions are prepared for storage by mixing the selected composition having the desired degree of purity with optional formulation agents (REMINGTON'S PHARMACEUTICAL SCIENCES, supra) in the form of a lyophilized cake or an aqueous solution. Further, in certain embodiments, the IL-17RA antigen binding protein product is formulated as a lyophilizate using appropriate excipients such as sucrose.

Pharmaceutical compositions can be selected for parenteral delivery. Alternatively, the compositions are selected for topical administration, for inhalation or for delivery through the digestive tract, such as orally. Preparation of such pharmaceutically acceptable compositions is within the skill of the art.

The formulation components are present preferably in concentrations that are acceptable to the site of administration. In certain embodiments, buffers are used to maintain the composition at physiological pH or at a slightly lower pH, typically within a pH range of from about 5 to about 8.

When parenteral administration is contemplated, the therapeutic compositions for use in this invention may be provided in the form of a pyrogen-free, parenterally acceptable aqueous solution comprising the desired IL-17RA antigen binding protein in a pharmaceutically acceptable vehicle. A particularly suitable vehicle for parenteral injection is sterile distilled water in which the IL-17RA antigen binding protein is formulated as a sterile, isotonic solution, properly preserved. In certain embodiments, the preparation involves the formulation of the desired molecule with an agent, such as injectable microspheres, bio-erodible particles, polymeric compounds (such as polylactic acid or polyglycolic acid), beads or liposomes, that provide controlled or sustained release of the product which is be delivered via depot injection.

Pharmaceutical compositions used for in vivo administration are typically provided as sterile preparations. Sterilization can be accomplished by filtration through sterile filtration membranes. When the composition is lyophilized, sterilization using this method is conducted either prior to or following lyophilization and reconstitution. Compositions for parenteral administration can be stored in lyophilized form or in a solution. Parenteral compositions generally are placed into a container having a sterile access port, for example, an intravenous solution bag or vial having a stopper pierceable by a hypodermic injection needle.

Herein disclosed are self-buffering IL-17RA antigen binding protein formulations, which can be used as pharmaceutical compositions, as described in international patent application WO 06138181A2. One embodiment provides self-buffering IL-17RA antigen binding protein formulations comprising an IL-17RA antigen binding protein in which the total salt concentration is less than 150 mM.

One embodiment provides IL-17RA antigen binding protein formulations comprising an IL-17RA antigen binding protein wherein the concentration of the IL-17RA antigen binding protein is between approximately: 20 and 400, or 20 and 300, or 20 and 250, or 20 and 200, or 20 and 150 mg/ml, optionally between approximately 20 and 400 mg/ml, optionally between approximately 20 and 250, and optionally between approximately 20 and 150 mg/ml.

One embodiment provides IL-17RA antigen binding protein formulations comprising an IL-17RA antigen binding protein wherein the pH maintained by the buffering action of the IL-17RA antigen binding protein is between approximately: 3.5 and 8.0, or 4.0 and 6.0, or 4.0 and 5.5, or 4.0 and 5.0, optionally between approximately 3.5 and 8.0, and optionally between approximately 4.0 and 5.5.

One embodiment provides IL-17RA antigen binding protein formulations comprising an IL-17RA antigen binding protein wherein the salt concentration is less than: 150 mM or 125 mM or 100 mM or 75 mM or 50 mM or 25 mM, optionally 150 mM, optionally 125 mM, optionally 100 mM, optionally 75 mM, optionally 50 mM, and optionally 25 mM.

One embodiment provides IL-17RA antigen binding protein formulations comprising an IL-17RA antigen binding protein and one or more pharmaceutically acceptable salts; polyols; surfactants; osmotic balancing agents; tonicity agents; antioxidants; antibiotics; antimycotics; bulking agents; lyoprotectants; anti-foaming agents; chelating agents; preservatives; colorants; analgesics; or additional pharmaceutical agents.

One embodiment provides IL-17RA antigen binding protein formulations comprising an IL-17RA antigen binding protein and one or more pharmaceutically acceptable polyols in an amount that is hypotonic, isotonic, or hypertonic, preferably approximately isotonic, particularly preferably isotonic, such as but not limited to any one or more of sorbitol, mannitol, sucrose, trehalose, or glycerol, optionally approximately 5% sorbitol, 5% mannitol, 9% sucrose, 9% trehalose, or 2.5% glycerol.

One embodiment provides IL-17RA antigen binding protein formulations comprising an IL-17RA antigen binding protein further comprising a surfactant, preferably one or more of polysorbate 20, polysorbate 80, other fatty acid esters of sorbitan, polyethoxylates, and poloxamer 188, preferably polysorbate 20 or polysorbate 80, optionally approximately 0.001 to 0.1 % polysorbate 20 or polysorbate 80, optionally approximately 0.002 to 0.02% polysorbate 20 or polysorbate 80, or optionally 0.002 to 0.02% polysorbate 20 or polysorbate 80.

One embodiment provides IL-17RA antigen binding protein formulations comprising an IL-17RA antigen binding protein wherein the formulation is sterile and suitable for treatment of a human or non-human subject.

As discussed above, certain embodiments provide IL-17RA antigen binding protein compositions, particularly pharmaceutical IL-17RA antigen binding protein compositions, that comprise, in addition to the IL-17RA antigen binding protein, one or more excipients such as those illustratively described in this section and elsewhere herein. Excipients can be used in the invention in this regard for a wide variety of purposes, such as adjusting physical, chemical, or biological properties of formulations, such as adjustment of viscosity, and or processes to improve effectiveness and or to stabilize such formulations and processes against degradation and spoilage due to, for instance, stresses that occur during manufacturing, shipping, storage, pre-use preparation, administration, and thereafter.

Embodiments of the IL-17RA antigen binding protein formulations further comprise one or more preservatives. Preservatives are necessary when developing multi-dose parenteral formulations that involve more than one extraction from the same container. Their primary function is to inhibit microbial growth and ensure product sterility throughout the shelf-life or term of use of the drug product. Commonly used preservatives include benzyl alcohol, phenol and m-cresol. Although preservatives have a long history of use with small-molecule parenterals, the development of protein formulations that includes preservatives can be challenging. Preservatives almost always have a destabilizing effect (aggregation) on proteins, and this has become a major factor in limiting their use in multi-dose protein formulations. To date, most protein drugs have been formulated for single-use only. However, when multi-dose formulations are possible, they have the added advantage of enabling patient convenience, and increased marketability. A good example is that of human growth hormone (hGH) where the development of preserved formulations has led to commercialization of more convenient, multi- use injection pen presentations. At least four such pen devices containing preserved formulations of hGH are currently available on the market. Norditropin® (liquid, Novo Nordisk), Nutropin AQ® (liquid, Genentech) & Genotropin (lyophilized- dual chamber cartridge, Pharmacia & Upjohn) contain phenol while Somatrope® (Eli Lilly) is formulated with m-cresol.

IL-17RA antigen binding protein formulations generally will be designed for specific routes and methods of administration, for specific administration dosages and frequencies of administration, for specific treatments of specific diseases, with ranges of bio-availability and persistence, among other things.

Formulations thus are designed in accordance with the invention for delivery by any suitable route, including but not limited to orally, aurally, opthalmically, rectally, and vaginally, and by parenteral routes, including intravenous and intraarterial injection, intramuscular injection, and subcutaneous injection. For example, a dose of the composition of the invention is delivered by subcutaneous injection by autoinjector syringe administered at time "0" (the first administration), at one week post time "0", and then administered every two weeks following the week one administration. In particular, an antibody or any other IL-17RA antigen binding protein can be used to treat nail or scalp psoriasis in adult and/or juvenile patients at a dose of 70 mg per dose delivered by subcutaneous injection by autoinjector syringe administered at time "0" (the first administration), at one week post time "0", and then administered every two weeks following the week one administration. An antibody or any other IL-17RA antigen binding protein can be used to treat nail or scalp psoriasis in adult and/or juvenile patients at a dose of 140 mg per dose delivered by subcutaneous injection by autoinjector syringe administered at time "0" (the first administration), at one week post time "0", and then administered every two weeks following the week one administration. An antibody or any other IL-17RA antigen binding protein can be used to treat nail or scalp psoriasis in adult and/or juvenile patients at a dose of 210 mg per dose delivered by subcutaneous injection by autoinjector syringe administered at time "0" (the first administration), at one week post time "0", and then administered every two weeks following the week one administration. An antibody or any other IL-17RA antigen binding protein can be used to treat nail or scalp psoriasis in adult and/or juvenile patients, and in particular plaque psoriasis, generalized pustular psoriasis and psoriatic erythroderma at a dose of 280 mg per dose delivered by subcutaneous injection by autoinjector syringe administered at time "0" (the first administration), at one week post time "0", and then administered every two weeks following the week one administration.

The therapeutically effective amount of an IL-17RA antigen binding protein-containing pharmaceutical composition to be employed will depend, for example, upon the therapeutic context and objectives. One skilled in the art will appreciate that the appropriate dosage levels for treatment will vary depending, in part, upon the molecule delivered, the indication for which the IL-17RA antigen binding protein is being used, the route of administration, and the size (body weight, body surface or organ size) and/or condition (the age and general health) of the patient. In certain embodiments, the clinician titers the dosage and modify the route of administration to obtain the optimal therapeutic effect. A typical dosage ranges from about 0.1 μg/kg to up to about 30 mg/kg or more, depending on the factors mentioned above. In specific embodiments, the dosage may range from 0.1 μg/kg up to about 30 mg/kg, optionally from 1 μg/kg up to about 30 mg/kg or from 10 μg/kg up to about 5 mg/kg.

Dosing frequency will depend upon the pharmacokinetic parameters of the particular IL-17RA antigen binding protein in the formulation used. Typically, a clinician administers the composition until a dosage is reached that achieves the desired effect. The composition is therefore administered as a single dose, or as two or more doses (which may or may not contain the same amount of the desired molecule) over time, or as a continuous infusion via an implantation device or catheter. In the treatment method of the present disclosure, an administration of the antibody or the antibody fragment thereof is not particularly limited, however, the administration is desirably performed at day 1, weeks 1 and 2 and may further be continued after week 2 every other week. Alternatively, continuous administrations may be performed at the starting date of the administration every other week. The dosing period of the antibody or the antibody fragment thereof is not particularly limited, however, the period is desirably 10 weeks or more, more desirably 50 weeks or more, further more desirably 62 weeks or more from the starting date of the administration. In addition, the dosing period may include a rest period. In the treatment method, the antibody or the antibody fragment thereof may be administered one time. Further refinement of the appropriate dosage is routinely made by those of ordinary skill in the art and is within the ambit of tasks routinely performed by them. Appropriate dosages are ascertained through use of appropriate dose-response data. In the treatment method or the therapeutic agent, a dose of the antibody or the antibody fragment thereof per one time is not particularly limited, however, is desirably 70 mg or more, 140 mg or more, 210 mg or more or 280 mg or more. Further, the dose may be increased or reduced during the continuous administration of the antibody or the antibody fragment thereof. In certain embodiments, the antigen binding proteins can be administered to patients throughout an extended time period. Chronic administration of an antigen binding protein minimizes the adverse immune or allergic response commonly associated with antigen binding proteins that are not fully human, for example an antibody raised against a human antigen in a non- human animal, for example, a non-fully human antibody or non-human antibody produced in a non-human species.

The route of administration of the pharmaceutical composition is in accord with known methods, e.g., orally, through injection by intravenous, intraperitoneal, intracerebral (intra-parenchymal), intracerebroventricular, intramuscular, intra-ocular, intraarterial, intraportal, or intralesional routes; by sustained release systems or by implantation devices. In certain embodiments, the compositions are administered by bolus injection or continuously by infusion, or by implantation device.

The composition also may be administered locally via implantation of a membrane, sponge or another appropriate material onto which the desired molecule has been absorbed or encapsulated. In certain embodiments, where an implantation device is used, the device is implanted into any suitable tissue or organ, and delivery of the desired molecule via diffusion, timed-release bolus, or continuous administration.

It also may be desirable to use IL-17RA antigen binding protein pharmaceutical compositions ex vivo. In such instances, cells, tissues or organs that have been removed from the patient are exposed to IL-17RA antigen binding protein pharmaceutical compositions after which the cells, tissues and/or organs are subsequently implanted back into the patient.

In particular, IL-17RA antigen binding proteins can be delivered by implanting certain cells that have been genetically engineered, using methods such as those described herein, to express and secrete the polypeptide. In certain embodiments, such cells, animal or human cells, and may be autologous, heterologous, or xenogeneic. In certain embodiments, the cells may be immortalized. In other embodiments, in order to decrease the chance of an immunological response, the cells may be encapsulated to avoid infiltration of surrounding tissues. In further embodiments, the encapsulation materials are typically biocompatible, semi- permeable polymeric enclosures or membranes that allow the release of the protein product(s) but prevent the destruction of the cells by the patient's immune system or by other detrimental factors from the surrounding tissues.

## EXAMPLES

The following example, including the experiments conducted and the results achieved, are provided for illustrative purposes only and are not to be construed as limiting the invention.

### Example 1

### Brodalumab Effectively Treats Nail and Scalp Psoriasis

#### Study Parameters and Overall Schema

Scalp and nail psoriasis were evaluated as part of the brodalumab phase 3 clinical trial which enrolled subjects with moderate to severe psoriasis. After a screening period, the study began with a 12-week double-blind, placebo-controlled induction phase. In this phase, the subjects were randomized in a 1:1:1 ratio to either received brodalumab at 210 mg Q2W, brodalumab at 140 mg Q2W or the placebo. Randomization was stratified by baseline total body weight (≤ 100kg; > 100 kg), by prior biologic use and by geographic region. Subjects with prior biologic use were capped at 50% of the study population. After 12 weeks, the treatment was withdrawn and retreatment was initiated with either the same dose or a placebo with the return of disease through 52 weeks. The study continued for 266 weeks. A summary of the overall study schema is provided in Figure 1.

Study subjects had stable moderate to severe plaque psoriasis for at least 6 months. Subjects were candidates to receive a biologic therapy for psoriasis, in the opinion of the investigator, according to regional labeling and had psoriasis that involves body surface area ≥ 10%, PASI ≥ 12, and sPGA ≥ 3 at screening and baseline visits. In particular, the subjects with a Psoriasis Scalp Severity Index (PSSI) ≥ 15 (out of a potential 72) and affected Scalp Surface Area (SSA) ≥ 30% at baseline were followed at scheduled visits. In subjects with nails involved with psoriasis, each nail was scored at baseline to determine the worst nail (i.e., the nail with the highest Nail Psoriasis Severity Index (NAPSI) score). In subjects whose worst nail has a minimum NAPSI score of 6 at baseline, that nail (the target nail) was followed for the remainder of the study.

#### Scalp Assessments

To determine the PSSI and SSA scores, assessments were completed by the same assessor performing the PASI assessment. The PSSI is a scalp-specific modification of the PASI, based on the extent of involvement and the severity of erthyema, induration and desquamation. The SSA numerical score (0% to 100%) measures the assessor's assessment of the proportion of the subject's total SSA involved with psoriasis. Those subjects with a PSSI ≥ 15 (out of a potential 72) and an SSA ≥ 30% at baseline were followed for these assessments at scheduled visits.

The scalp psoriasis PSSI- 75 (NRI) and the PSSI-100 were measured every two weeks. The PSSI-75 or PSSI-100 refers to that percent of patients who achieved 75% or 100% of the PSSI percent improvement from baseline. As shown in Figures 2 and 3, brodalumab at doses140 mg and 210 mg Q2W is more efficacious than placebo at treating scalp psoriasis as measured by the PSSI and SSA at week 12. The PSSI (MI) percent improvement from baseline is provided in Figure 4.

#### Nail Assessments

The NAPSI scale is objective, numeric and reproducible grading system for nail psoriasis that incorporates the many different features of nail psoriasis. For assessments in the study (including selection of target nail), a nail was graded using the NAPSI scale by first dividing the nail with imaginary horizontal and vertical lines into 4 quarters. The following eight clinical features of nail psoriasis are then scored based on the number of quarters in which the feature is present (0 to 4) to arrive at a NAPSI score of 0 to 32 for each nail: pitting, leukonychia, red spots in lunula, nail plate crumbling, oil drop (salmon patch) discoloration, onycholysis, nail bed hyperkeratosis, splinter hemorrhages.

In randomized subjects with nails involving psoriasis, each nail was scored at baseline to determine the worst nail (i.e., the nail with the highest NAPSI score). Those subjects whose nail has a minimum NAPSI score of 6 at baseline will have this nail (the target nail) followed for the remainder of the study. If multiple nails have the same worst score, only 1 target nail was followed.

The NAPSI score (as observed) by treatment group in the induction phase is provided in Figure 5. The NAPSI score (as observed) for non-rerandomized subjects by treatment group in the induction phase through week 52 is provided in Figure 6. Brodalumab at dose 140 mg and 210 mg Q2W was more efficacious than placebo at treating nail psoriasis as measured by the NAPSI at week 12. Brodalumab at doses 140 mg and 210 mg Q2W was effective at treating nail psoriasis as measured by the NAPSI over 52 weeks.

## Adverse Events

The subject incidence rates for the placebo-controlled 12 week induction phase in the overall study population are set out in Table 1 below. The most frequently reported adverse event (occurring in ≥5% in any treatment group) were nasopharyngitis, upper respiratory tract infection, and headache. There were no meaningful imbalances in the AE rates in the induction phase between placebo and brodalumab treatment arms.

**Table 1**

| | | Brodalumab | | | |
|---|---|---|---|---|---|
| | | **Placebo** | **140 mg Q2W** | **210mg Q2W** | **All** |
| | | **(N = 220)** | **(N = 219)** | **(N=222)** | **(N = 441)** |
| All treatment-emergent adverse events - n (%) | | 112 (50.9) | 126 (57.5) | 131 | 257 (58.3) |
| Grade ≥ 2 | | 62 (28.2) | 70 (32.0) | 75 (33.8) | 145 (32.9) |
| Grade ≥ 3 | | 9 (4.1) | 8 (3.7) | 15 (6.8) | 23 (5.2) |
| Serious | | 3 (1.4) | 6 (2.7) | 4 (1.8) | 10 (2.3) |
| Fatal adverse events | | 0 (0.0) | 0 (0.0) | 0 (0.0) | 0 (0.0) |

The exposure adjusted event rates through week 52 in the overall study population are set out in Table 2 below. The term "Subj-yr" refers to the total subject years of exposure through week 52 excluding periods of placebo exposure in the induction or withdrawal phases. The term "n" refers to the number of adverse events reported. The term "r" refers to exposure-adjusted event rate per 100 subject-years (n/subj-yr*100). Adverse events reported during periods of placebo exposure were excluded. The adverse events and exposure in subjects randomized to placebo in the induction phase were summarized after their first dose of 210 mg Q2W at or after week 12 under the constant 210 mg Q2W dose group. Multiple occurrences of the same event for a subject were counted as multiple events.

Treatment groups are defined as planned through week 52. Constant Dose groups are subjects who received the same dose across all phases after first brodalumab administration. The Combination of 140/210 groups are those subjects with planned treatment with both 140 mg Q2W and 210 mg Q2W through week 52. The Mixed Dosing group refers to subjects that were re-randomized to placebo in withdrawal period.

**Table 2**

| | | Brodalumab | | | | |
|---|---|---|---|---|---|---|
| | | **Mixed dosing (Subj-yr = 105.2)** | **Combination 140/210 mg Q2W (Subj-yr = 89.1)** | **Constant 140 mg Q2W (Subj-yr = 51.2)** | **Constant 210 mg Q2W (Subj-yr = 271.8)** | **All (Subj-yr = 517.3)** |
| | | **(N = 143)**<br>**n (r)** | **(N = 99)**<br>**n (r)** | **(N = 61)**<br>**n (r)** | **(N = 345)**<br>**n (r)** | **(N=648)**<br>**n (r)** |
| All treatment-emergent adverse events - n (%) | | 343 (326.1) | 347 (389.2) | 184 (359.2) | 1034 (380.4) | 1908 (368.8) |
| Grade ≥ 2 | | 140 (133.1) | 167 (187.3) | 79 (154.2) | 460 (169.2) | 846 (163.5) |
| Grade ≥ 3 | | 12 (11.4) | 13 (14.6) | 11 (21.5) | 55 (20.2) | 91 (17.6) |
| Serious | | | | 5 | 27 (9.9) | |

(continued)

| | | Brodalumab | | | | |
|---|---|---|---|---|---|---|
| | | Mixed dosing (Subj-yr = 105.2) | Combination 140/210 mg Q2W (Subj-yr = 89.1) | Constant 140 mg Q2W (Subj-yr = 51.2) | Constant 210 mg Q2W (Subj-yr = 271.8) | All (Subj-yr = 517.3) |
| | | (N = 143) n (r) | (N = 99) n (r) | (N = 61) n (r) | (N = 345) n (r) | (N=648) n (r) |
| Leading to discontinuation of IP | | 1 (1.0) | 1 (1.1) | 4 (7.8) | 10 (3.7) | 16(3.1) |
| Leading to discontinuation from study | | 1(1.0) | 1 (1.1) | 3(5.9) | 9 (3.3) | 14(2.7) |
| Fatal adverse events | | 1(1.0) | 0 (0.0) | 0 (0.0) | 3(1.1) | 4(0.8) |

**Example 2**

**Phase II Clinical Trial of Brodalumab in Japan**

A randomized, double blind, placebo-controlled phase II clinical trial was carried out in Japan to investigate the effectiveness of the human monoclonal AM-14 (known an brodalumab) in subjects with moderate to severe plaque psoriasis. 151 subjects suffering from moderate to severe psoriasis, which satisfied the conditions provided in Table 3, were randomly divided into four groups. The subjects in each group were administered one of the following dose of brodalumab: 0 mg (placebo), 70 mg, 140 mg or 210 mg. Brodalumab was administered as a subcutaneous injection on day 1 and at week 1, week 2, week 4, week 6, week 8 and week 10.

**Table 3**

| | |
|---|---|
| Ages Eligible for Study: 20 Years to 70 Years | |
| Genders Eligible for Study: | Both |
| Accepts Healthy Volunteers: | No |
| Criteria<br>1) Inclusion Criteria:<br>• Subject had stable moderate to severe plaque psoriasis for at least 6 months.<br>• Subject received at least one previous phototherapy or systemic psoriasis therapy or has been a candidate to receive phototherapy or systemic psoriasis therapy in the opinion of the investigator.<br>• Subject has involved BSA ≥ 10% and PASI ≥ 12 at screening and at baseline.<br><br>2) Exclusion Criteria:<br>• Subject diagnosed with erythrodermic psoriasis, pustular psoriasis, medication-induced, or medication-exacerbated psoriasis.<br>• Evidence of skin conditions at the time of the screening visit (eg, eczema) that would interfere with evaluations of the effect of investigational product on psoriasis.<br>• Subject had any active Common Terminology Criteria for Adverse Events (CTCAE) grade 2 or higher infection<br>• Subject a significant concurrent medical condition or laboratory abnormalities, as defined in the study protocol.<br>• Subject used the following therapies within 14 days of the first dose: topical calcineurin inhibitors including tacrolimus, topical vitamin A, activated form D3 or activated form D3 analogue preparations, weak through strong topical steroids (excluding application on the scalp, axillae, and groin) | |

(continued)

| |
|---|
| • Subject used the following therapies within 28 days of the first dose: any other systemic psoriasis therapy (eg, vitamin A, calcineurin inhibitors, methotrexates, steroids), UVA therapy (with or without psoralen), very strong or strongest topical steroid, tar therapy<br>• Subject used the following therapies within 3 months of the first dose: adalimumab, etanercept, infliximab, or live vaccines<br>• Subject used ustekinumab within 6 months of the first dose<br>• Subject previously used an anti-interleukin-17 biologic therapy |

The degree of severity of psoriasis symptoms on the nail and the scalp of the subjects in each administered group was measured on the first day brodalumab was administered to the subject (hereinafter, described as week 0 from the first administration day 1 from the first administration), Week 0 or day 1) and at week 12 after the first administration. The NAPSI and PSSI score were respectively calculated based on the descriptions by Rich et al. (J Am Acad Dermatol; vol. 49, number 2, p. 206-212) and Leonardi et al. (J Engl J Med 2012; 366: 1190-9).

Figure 7 provides the mean percent change in the NAPSI (panel A) and PSSI (panel B) from baseline and demonstrates that brodalumab had a therapeutic effect on nail (Fig. 7A) and scalp psoriasis (Fig. 7B). The mean percent change from baseline is calculated according to formula 1 and the baseline set as day 1 of this trial in this analysis. The obtained mean percent change in the NAPSI score in the placebo administered group was a reduction by 9.6%, while the mean percent change in the NAPSI scores from baseline was a reduction by 9.1%, 44.9% and 47.3% in response to 70 mg 140 mg and 210 mg of brodalumab, respectively. Thus, the NAPSI scores were greatly reduced in the subjects receiving 140 mg and 210 mg of brodalumab as compared to the placebo group.

In addition, a mean percent change in the PSSI score from baseline in the placebo administered group was reduced by 12.6%, while the men percent change in the PSSI from baseline was a reduction by 38.3%, 73.8% and 94.5% in response to 70 mg 140 mg and 210 mg of brodalumab, respectively. Thus, the PSSI scores were greatly reduced in the subjects receiving 70 mg, 140 and 210 mg of brodalumab as compared to the placebo group. This trial demonstrated that brodalumab improved the degree of severity of nail and scalp psoriasis in subjects suffering from moderate to severe plaque psoriasis.

## Example 3

### Phase III Clinical Trial of Brodalumab in Japan

A randomized, placebo-controlled, randomized phase III clinical trial was carried out in Japan to investigate the safety and effectiveness of the human monoclonal antibody AM-14 (known an brodalumab). The trial was double-blind and randomized for 4 week after the start of administration of brodalumab, followed by an open-labeled, randomized extension study from week 4 to week 52. The subjects suffering from moderate to severe plaque psoriasis that completed the trial described in Example 2 and satisfied the conditions presented in Table 4 were selected as subjects for this phase III trial. This phase III study was started on the date of the completion of the phase II trial (Example 2).

145 subjects were enrolled in the trial (133 subjects completed the trial) and were divided into 2 groups. The subjects receiving 140 mg or 210 mg in the phase II trial were administered the same amount of brodalumab by subcutaneous injection every other week in the phase III trial. The subjects administered with 0 mg (placebo) or 70 mg of AM-14 in the phase II study were randomly divided in two groups in the phase III trial, and each group was administered with 140 mg or 210 mg of AM-14 in weeks 0, 1, and 2. After week 2, the antibody was administered every other week. AM-14 administration was continued for 50 weeks, and Phase II trial was completed on week 52.

**Table 4**

| | |
|---|---|
| Ages Eligible for Study: 20 Years to 70 Years | |
| Genders Eligible for Study: | Both |
| Accepts Healthy Volunteers: | No |
| Criteria<br>Inclusion Criteria:<br>• Subject has voluntarily signed the written informed consent form to participate in this study<br>• Subject has completed the week 12 evaluation of Phase II trial (Example 2) | |

(continued)

| Exclusion Criteria: |
|---|
| • Subject has had a serious infection, defined as requiring systemic treatment with antibiotics or antivirals (excluding oral administration) |
| • Subject had been judged to be ineligible for participation in the study by the investigators/subinvestigators |

The degree of severity of psoriasis symptoms on the nail and the scalp of each subject was measured on day 1 of phase II trial (Example 2) and at week 52 of the phase III trial. NAPSI and PSSI scores were calculated in the same manner as described in Example 2.

Table 5 and Table 6 showed the mean percent change in NAPSI and PSSI score from baseline, respectively. The mean percent change from baseline was calculated according to formula 1. In this analysis, day 1 of the Phase II trial (Example 2) was set as baseline.

**Table 5 The mean percent change in NAPSI score from baseline**

| | Phase II | Phase III | | | | |
|---|---|---|---|---|---|---|
| | Day 1 (baseline) | Week 0 | Week 12 | Week 24 | Week 36 | Week 52 |
| 140 mg of brodalumab (N = 43) | 0% | -29.7% | -51.7% | -74.8% | -73.1% | -73.7% |
| 210 mg of brodalumab (N = 46) | 0% | -26.4% | -73.0% | -85.4% | -87.7% | -88.1% |

**Table 6 The mean percent change in PSSI score from baseline**

| | Phase II | Phase III | | | | |
|---|---|---|---|---|---|---|
| | Day 1 (baseline) | Week 0 | Week 12 | Week 24 | Week 36 | Week 52 |
| 140 mg of brodalumab (N = 68) | 0% | -51.4% | -86.4% | -83.8% | -82.4% | -84.6% |
| 210 mg of brodalumab (N = 69) | 0% | -60.4% | -90.4% | -87.4% | -82.8% | -82.6% |

Thus, the NAPSI and PSSI score greatly decreased from day 1 of the phase II trial to the date of completion of the phase III trial in subjects receiving 140 mg and 210 mg of brodalumab. The degree of severity of the nail and scalp psoriasis greatly improved in plaque psoriasis patients administered with brodalumab.

**Example 4**

**Phase III Clinical Trial of Brodalumab in Japan**

Another Phase III Clinical Trial of brodalumab was conducted in Japan in subjects with generalized pustular psoriasis (GPP) or psoriatic erythroderma (PsE). A summary of this clinical trials was described in Table 7.

**Table 7**

| Indication, Design | Dose, Duration | No. Subjects Enrolled |
|---|---|---|
| ▪ Indication; generalized pustular psoriasis (GPP) or psoriatic erythroderma (PsE)<br>▪ Design; Open-label, Uncontrolled Study | ▪ Dose; 140 mg (dosage may be increased to 210 mg in case of insufficient efficacy)<br>▪ Duration; 50 weeks | ▪ 12 patients (GPP),<br>▪ 18 patients (PsE) |

Pustular psoriasis patients or psoriatic erythroderma patients that satisfy the conditions presented in Table 8 were selected as clinical trial subjects, and evaluated for the safety and efficacy or the like of brodalumab after chronic administration. Each subject was administered with 140 mg of brodalumab on day 1, in week 1 and in week 2, and every other week from week 2 to week 50. For subjects in which a sufficient effect was not obtained by the end of week 4, the dose was increased and 210 mg of AM-14 was administered every other week from week 6.

**Table 8**

| |
|---|
| Ages Eligible for Study: 18 Years and older |
| Genders Eligible for Study: Both |
| Accepts Healthy Volunteers: No |
| Criteria<br>1)Inclusion Criteria:<br>•Subject has signed voluntarily the written informed consent form to participate in this study.<br>•Subject has been diagnosed as pustular psoriasis or psoriatic erythroderma.<br>•Subject has received at least one previous phototherapy or systemic psoriasis therapy or has been a candidate to receive phototherapy or systemic psoriasis therapy in the opinion of the investigator.<br><br>2) Exclusion Criteria:<br>•Subject with psoriatic erythroderma has involved Body surface area (BSA) of lesion < 80% at baseline.<br>•Subject diagnosed with guttate psoriasis, medication-induced or medication-exacerbated psoriasis.<br>•Evidence of skin conditions at the time of the screening visit (eg, eczema) that would interfere with evaluations of the effect of AM-14 on psoriasis.<br>•Subject has any active Common Terminology Criteria for Adverse Events (CTCAE) grade 2 or higher infection<br>•Subject has a significant concurrent medical condition or laboratory abnormalities, as defined in the study protocol.<br>•Subject has used Ultra Violet B (UVB) therapy within 14 days of the first dose or Ultra Violet A (UVA) (with or without psoralen) within 28 days of the first dose.<br>•Subject has used etanercept, adalimumab, infliximab or ustekinumab within 1 week, 2 weeks, 8 weeks or 12 weeks of the first dose, respectively.<br>•Subject has stopped ustekinumab or other anti-Interleukin (IL)-23 biologics therapy due to lack of efficacy<br>•Subject has used live vaccine within 3 months of the first dose<br>•Subject has previously used an anti-IL-17 biologic therapy |

The degree of severity of psoriasis sympton on the nail and the scalp of each subject was measured on week 0, 12, 24, 36, 52 of this phase III trial. NAPSI and PSSI score were calculated in the same manner as described in Example 2.

Table 9 and Table 10 showed the mean percent change in NAPSI and PSSI score from baseline, respectively. The mean percent change was calculated according to formula 1. In this analysis Day 1(Week 0) of this trial was set as baseline.

**Table 9 The mean percent change in NAPSI score from baseline**

| | Week0 (baseline) | Week12 | Week24 | Week36 | Week52 |
|---|---|---|---|---|---|
| Pustular (N = 4) | 0% | -47.6% | -59.2% | -60.7% | -67.5% |
| Erythroderma (N = 13) | 0% | -45.5% | -66.3% | -83.9% | -83.5% |

**Table 10 The mean percent change in PSSI score from baseline**

| | Week0 (baseline) | Week12 | Week24 | Week36 | Week52 |
|---|---|---|---|---|---|
| Pustular (N = 9) | 0% | -77.7% | -87.5% | -95.2% | -96.1% |
| Erythroderma (N = 18) | 0% | -82.5% | -89.9% | -96.2% | -95.9% |

Thus, the NAPSI and PSSI score was greatly reduced in subjects receiving brodalumab. This trial demonstrated that brodalumab improved the degree of severity of nail and scalp psoriasis in subjects suffering from GPP or PsE.

## Claims

1. A composition for use in the treatment of nail or scalp psoriasis in a patient suffering from psoriasis, wherein the composition comprises an antibody or fragment thereof that specifically binds to IL-17 Receptor A (IL-17RA) and has an antagonistic activity,

   wherein the antibody comprises a light chain CDR1 comprising the amino acid sequence of SEQ ID NO:1, a light chain CDR2 comprising the amino acid sequence of SEQ ID NO: 2, a light chain CDR3 comprising the amino acid sequence of SEQ ID NO: 3, a heavy chain CDR1 comprising the amino acid sequence of SEQ ID NO:4, a heavy chain CDR2 comprising the amino acid sequence of SEQ ID NO:5, and a heavy chain CDR3 comprising the amino acid sequence of SEQ ID NO:6,
   and wherein the patient has a Nail Psoriasis Severity Index (NAPSI) score of at least 6 on one or more nails and/or a Psoriasis Scalp Severity Index (PSSI) score of at least 15.

2. The composition for use of claim 1, wherein the antibody or fragment thereof is a humanized antibody or a chimeric antibody.

3. The composition for use of claim 1, wherein the antibody or fragment thereof is an antibody, comprising a light chain variable domain comprising the amino acid sequence of SEQ ID NO:7 and a heavy chain variable domain comprising the amino acid sequence of SEQ ID NO:8, optionally wherein the antibody is a chimeric antibody.

4. The composition for use of claim 1 or claim 2, wherein said antibody is selected from the group consisting of:

   a. an antibody comprising the full length light chain amino acid sequence of SEQ ID NO: 9 and a full length heavy chain amino acid sequence of SEQ ID NO: 10;
b. a monoclonal antibody;
c. an antigen-binding antibody fragment;
d. a single chain antibody;
e. a diabody;
f. a triabody;
g. a tetrabody;
h. a Fab fragment;
i. a F(ab')2 fragment;
j. an IgD antibody;
k. an IgE antibody;
l. an IgM antibody;
m. an IgG1 antibody;
n. an IgG2 antibody;
o. an IgG3 antibody; and
p.an IgG4 antibody.

**5.** The composition for use of any one of claims 1, 3 and 4, wherein said antibody is a human IgG2 monoclonal antibody.

**6.** The composition for use of any one of claims 1-5, wherein the antibody or fragment thereof inhibits binding of IL-17A to said IL-17RA.

**7.** The composition for use of claim 6, wherein the patient has about 7% or less, about 5% or less, about 3% or less, or about 1% or less body surface area affected by psoriasis.

**8.** The composition for use of any one of claims 1-3, wherein the patient is suffering from plaque psoriasis, pustular psoriasis or psoriatic erythroderma.

**9.** The composition for use of claim 8, wherein:

(a) the patient has:

(i) a modified NAPSI score of at least 2 or 3 on one or more nails, and/or
(ii) a Scalp Surface Area (SSA) score of at least 30%, and/or
(iii) less than 10% body surface area affected by psoriasis and additionally has nail or scalp psoriasis, and/or
(iv) less than 10% body surface area affected by moderate to severe psoriasis, plaque psoriasis, pustular psoriasis or psoriatic erythroderma, and/or

(b) the composition comprises a dose of antibody that is 70 mg, 140mg, 210 mg or 280 mg.

**10.** The composition for use of any one of claims 1-9, wherein the treatment comprises administering the composition with a second treatment.

**11.** The composition for use of claim 10, wherein said second treatment is administered prior to, concurrent with, or subsequent to administration of said medicament or composition comprising said antibody.

**12.** The composition for use of claim 10 or claim 11, wherein the second treatment is a topical treatment, optionally wherein the topical treatment is selected from the group consisting fluorouracil, dithranol, tazarotene, cyclosporine, calcineneurin inhibitors, triamcinolone, fluocinonide, topical steroids, vitamin D3, vitamin D3 analogs, betamethasone dipropionate, betamethasone valerate, calcipotriol, clobetasol, XAMIOL, DAIVOBET, coal tar, urea, corticosteroids, retinoids, anthralin, topical methatrexate, keratolytics, salicylic acid, tofacitinab, apremilast, topical JAK inhibitors or a combination thereof.

**13.** The composition for use of any one of claims 10-12, wherein the second treatment is selected from the group consisting of retinoids, acitretin cyclosporine, methotrexate, apremilast, tofacitinib, oral JAK inhibitors, oral PI3 kinase inhibitors, oral MAP kinase inhibitors, Fumaderm, fumarates, dimethyl fumarate, sulfasalazine, leflunomide, calcineurin inhibitors, azathioprine, thioguanine, hydroxyurea, hydroxychloroquine, sulfasalazine, antifungals or a combination thereof.

**14.** The composition for use of any one of claims 10-13, wherein the second treatment is:

(a) an antibody or chimeric protein specific for TNF, IL-17, IL-12/23 or IL-23, optionally wherein the antibody or chimeric protein is infliximab, adalimumab, etanercept, alefacept, ustekinumab, secukinumab, ixekizumab, guselkumab, antifungals or a combination thereof, and/or
(b) selected from the group consisting of triamcinolone acetonide photochemotherapy, laser therapy, Excimer laser, oral/topical psorallen with UVA (PUV A), pulsed dye laser, radiation therapy, superficial radiotherapy, electron beam therapy, Grenz ray therapy, ermatome shaving, aloe vera extract, narrow band UV therapy, UV therapy or a combination thereof.

**15.** The composition for use of any one of claims 1-14, wherein said composition further comprises a pharmaceutically acceptable diluent.

**16.** The composition for use of any one of claims 1-15, wherein said composition comprises an aqueous solution of a glutamic acid buffer, and wherein a) said glutamic acid buffer comprises a glutamic acid concentration of 5-30 mM$\pm$0.2 mM; b) said glutamic acid buffer comprises a pH of 4.5-5.2$\pm$0.2; c) said formulation further comprises 2-4% proline (w/v) and 0.005-0.02% (w/v) polysorbate 20 and d) the antibody or fragment thereof is at a concentration of 100 to 150 mg/ml.

**17.** The composition for use of claim 16, wherein the composition has:

(a) an osmolarity of 275 to 325 osm, and/or
(b) a viscosity of 5 to 7 cP at 25°C.

**Patentansprüche**

**1.** Zusammensetzung zur Verwendung bei der Behandlung von Nagel- oder Kopfhautpsoriasis bei einem an Psoriasis leidenden Patienten, wobei die Zusammensetzung einen Antikörper oder ein Fragment davon, der/das spezifisch an den IL-17-Rezeptor A (IL-17RA) bindet und antagonistische Wirkung hat, umfasst,
wobei der Antikörper eine Leichte-Kette-CDR1 mit der Aminosäuresequenz von SEQ ID NO: 1, eine Leichte-Kette-CDR2 mit der Aminosäuresequenz von SEQ ID NO: 2, eine Leichte-Kette-CDR3 mit der Aminosäuresequenz von SEQ ID NO: 3, eine Schwere-Kette-CDR1 mit der Aminosäuresequenz von SEQ ID NO: 4, eine Schwere-Kette-CDR2 mit der Aminosäuresequenz von SEQ ID NO: 5 und eine Schwere-Kette-CDR3 mit der Aminosäuresequenz von SEQ ID NO: 6 umfasst,
und wobei der Patient eine Nail Psoriasis Severity Index(NAPSI)-Bewertung von mindestens 6 an einem oder mehreren Nägeln und/oder eine Psoriasis Scalp Severity Index(PSSI)-Bewertung von mindestens 15 hat.

**2.** Zusammensetzung zur Verwendung nach Anspruch 1, wobei es sich bei dem Antikörper oder dem Fragment davon um einen humanisierten Antikörper oder einen chimären Antikörper handelt.

**3.** Zusammensetzung zur Verwendung nach Anspruch 1, wobei es sich bei dem Antikörper oder dem Fragment davon um einen Antikörper handelt, der eine variable Leichte-Kette-Domäne mit der Aminosäuresequenz von SEQ ID NO: 7 und eine variable Schwere-Kette-Domäne mit der Aminosäuresequenz von SEQ ID NO: 8 umfasst, wobei es sich bei dem Antikörper gegebenenfalls um einen chimären Antikörper handelt.

**4.** Zusammensetzung zur Verwendung nach Anspruch 1 oder Anspruch 2, wobei der Antikörper aus der folgenden Gruppe ausgewählt ist:

a. einem Antikörper mit der vollständigen Leichte-Kette-Aminosäuresequenz von SEQ ID NO: 9 und einer vollständigen Schwere-Kette-Aminosäuresequenz von SEQ ID NO: 10,
b. einem monoklonalen Antikörper,
c. einem antigenbindenden Antikörperfragment,
d. einem Einzelkettenantikörper,
e. einem Diabody,
f. einem Triabody,
g. einem Tetrabody,
h. einem Fab-Fragment,

i. einem F(ab')2-Fragment,
j. einem IgD-Antikörper,
k. einem IgE-Antikörper,
l. einem IgM-Antikörper,
m. einem IgG1-Antikörper,
n. einem IgG2-Antikörper,
o. einem IgG3-Antikörper und
p. einem IgG4-Antikörper.

**5.** Zusammensetzung zur Verwendung nach einem der Ansprüche 1, 3 und 4, wobei es sich bei dem Antikörper um einen humanen monoklonalen IgG2-Antikörper handelt.

**6.** Zusammensetzung zur Verwendung nach einem der Ansprüche 1-5, wobei der Antikörper oder das Fragment davon die Bindung von IL-17A an den IL-17RA inhibiert.

**7.** Zusammensetzung zur Verwendung nach Anspruch 6, wobei bei dem Patienten etwa 7% oder weniger, etwa 5% oder weniger, etwa 3% oder weniger oder etwa 1% oder weniger der Körperoberfläche von Psoriasis betroffen ist.

**8.** Zusammensetzung zur Verwendung nach einem der Ansprüche 1-3, wobei der Patient an Plaquepsoriasis, pustulöser Psoriasis oder psoriatischer Erythrodermie leidet.

**9.** Zusammensetzung zur Verwendung nach Anspruch 8, wobei

(a)

(i) der Patient an einem oder mehreren Nägeln eine modifizierte NAPSI-Bewertung von mindestens 2 oder 3 hat und/oder
(ii) der Patient eine Scalp Surface Area(SSA)-Bewertung von mindestens 30% hat und/oder
(iii) weniger als 10% der Körperoberfläche des Patienten von Psoriasis betroffen ist und der Patient zusätzlich an Nagel- oder Kopfhautpsoriasis leidet und/oder
(iv) weniger als 10% der Körperoberfläche des Patienten von moderater bis schwerer Psoriasis, Plaquepsoriasis, pustulöser Psoriasis oder psoriatischer Erythrodermie betroffen ist und/oder

(b) die Zusammensetzung eine Antikörperdosis von 70 mg, 140 mg, 210 mg oder 280 mg umfasst.

**10.** Zusammensetzung zur Verwendung nach einem der Ansprüche 1-9, wobei die Behandlung die Verabreichung der Zusammensetzung mit einer zweiten Behandlung umfasst.

**11.** Zusammensetzung zur Verwendung nach Anspruch 10, wobei die zweite Behandlung vor der Verabreichung des den Antikörper umfassenden Medikaments bzw. der den Antikörper umfassenden Zusammensetzung oder gleichzeitig damit oder im Anschluss daran verabreicht wird.

**12.** Zusammensetzung zur Verwendung nach Anspruch 10 oder Anspruch 11, wobei es sich bei der zweiten Behandlung um eine topische Behandlung handelt, wobei die topische Behandlung gegebenenfalls ausgewählt ist aus der Gruppe bestehend aus Fluoruracil, Dithranol, Tazaroten, Cyclosporin, Calcineurininhibitoren, Triamcinolon, Fluocinonid, topischen Steroiden, Vitamin D3, Vitamin-D3-Analoga, Betamethasondipropionat, Betamethasonvalerat, Calcipotriol, Clobetasol, XAMIOL, DAIVOBET, Kohlenteer, Harnstoff, Corticosteroiden, Retinoiden, Anthralin, topischem Methatrexat, Keratolytika, Salicylsäure, Tofacitinab, Apremilast, topischen JAK-Inhibitoren oder einer Kombination davon.

**13.** Zusammensetzung zur Verwendung nach einem der Ansprüche 10-12, wobei die zweite Behandlung ausgewählt ist aus der Gruppe bestehend aus Retinoiden, Acitretin, Cyclosporin, Methotrexat, Apremilast, Tofacitinib, oralen JAK-Inhibitoren, oralen PI3-Kinase-Inhibitoren, oralen MAP-Kinase-Inhibitoren, Fumaderm, Fumaraten, Dimethylfumarat, Sulfasalazin, Leflunomid, Calcineurininhibitoren, Azathioprin, Thioguanin, Hydroxyharnstoff, Hydroxychloroquin, Sulfasalazin, Antipilzmitteln oder einer Kombination davon.

**14.** Zusammensetzung zur Verwendung nach einem der Ansprüche 10-13, wobei:

(a) es sich bei der zweiten Behandlung um einen Antikörper oder ein chimäres Protein spezifisch für TNF, IL-17, IL-12/23 oder IL-23 handelt, wobei es sich bei dem Antikörper bzw. dem chimären Protein gegebenenfalls um Infliximab, Adalimumab, Etanercept, Alefacept, Ustekinumab, Secukinumab, Ixekizumab, Guselkumab, Antipilzmittel oder eine Kombination davon handelt, und/oder
(b) die zweite Behandlung aus der aus Triamcinolonacetonidphotochemotherapie, Lasertherapie, Excimerlaser, oralem/topischem Psoralen mit UVA (PUV A), gepulstem Farbstofflaser, Strahlentherapie, oberflächlicher Strahlentherapie, Elektronenstrahltherapie, Grenz-Strahlentherapie, Eramtomschälen, Aloe-Vera-Extrakt, Schmalband-UV-Therapie, UV-Therapie oder einer Kombination davon bestehenden Gruppe ausgewählt ist.

**15.** Zusammensetzung zur Verwendung nach einem der Ansprüche 1-14, wobei die Zusammensetzung weiterhin ein pharmazeutisch unbedenkliches Verdünnungsmittel umfasst.

**16.** Zusammensetzung zur Verwendung nach einem der Ansprüche 1-15, wobei die Zusammensetzung eine wässrige Lösung eines Glutaminsäurepuffers umfasst und wobei a) der Glutaminsäurepuffer eine Glutaminsäurekonzentration von 5-30 mM$\pm$0,2 mM umfasst, b) der Glutaminsäurepuffer einen pH-Wert von 4,5-5,2$\pm$0,2 aufweist, c) die Formulierung weiterhin 2-4% Prolin (w/v) und 0,005-0,02% (w/v) Polysorbat 20 umfasst und d) der Antikörper oder das Fragment davon in einer Konzentration von 100 bis 150 mg/ml vorliegt.

**17.** Zusammensetzung zur Verwendung nach Anspruch 16, wobei die Zusammensetzung:

(a) eine Osmolarität von 275 bis 325 osm aufweist und/oder
(b) bei 25°C eine Viskosität von 5 bis 7 cP aufweist.

## Revendications

**1.** Composition destinée à une utilisation dans le traitement du psoriasis des ongles ou du cuir chevelu chez un patient souffrant de psoriasis, **caractérisée en ce que** la composition comprend un anticorps ou un fragment de celui-ci qui se fixe spécifiquement au Récepteur A d'IL-17 (IL-17RA) et possède une activité antagoniste,
où l'anticorps comprend une chaîne légère CDR1 comprenant la séquence d'acides aminés de SEQ ID n° 1, une chaîne légère CDR2 comprenant la séquence d'acides aminés de SEQ ID n° 2, une chaîne légère CDR3 comprenant la séquence d'acides aminés de SEQ ID n° 3, une chaîne lourde CDR1 comprenant la séquence d'acides aminés de SEQ ID n° 4, une chaîne lourde CDR2 comprenant la séquence d'acides aminés de SEQ ID n° 5, et une chaîne lourde CDR3 comprenant la séquence d'acides aminés de SEQ ID n° 6,
et où le patient possède un score d'Indice de Gravité du Psoriasis des Ongles (NAPSI) d'au moins 6 sur un ou plusieurs ongles et/ou un score d'Indice de Gravité du Psoriasis du Cuir Chevelu (PSSI) d'au moins 15.

**2.** Composition destinée à une utilisation selon la revendication 1, **caractérisée en ce que** l'anticorps ou un fragment de celui-ci est un anticorps humanisé ou un anticorps chimère.

**3.** Composition destinée à une utilisation selon la revendication 1, **caractérisée en ce que** l'anticorps ou un fragment de celui-ci est un anticorps comprenant un domaine variable de chaîne légère comprenant la séquence d'acides aminés de SEQ ID n° 7 et un domaine variable de chaîne lourde comprenant la séquence d'acides aminés de SEQ ID n° 8, éventuellement où l'anticorps est un anticorps chimère.

**4.** Composition destinée à une utilisation selon la revendication 1 ou la revendication 2, **caractérisée en ce que** ledit anticorps est choisi dans le groupe constitué par :

a. un anticorps comprenant la séquence d'acides aminés pleine longueur de chaîne légère de SEQ ID n° 9 et la séquence d'acides aminés pleine longueur de chaîne lourde de SEQ ID n° 10 ;
b. un anticorps monoclonal ;
c. un fragment d'anticorps fixant l'antigène ;
d. un anticorps à chaîne unique ;
e. un di-anticorps ;
f. un tri-anticorps ;
g. un tétra-anticorps ;
h. un fragment Fab ;
i. un fragment F(ab')2 ;

j. un anticorps IgD ;
k. un anticorps IgE ;
l. un anticorps IgM ;
m. un anticorps IgG1 ;
n. un anticorps IgG2 ;
o. un anticorps IgG3 ;
p. un anticorps IgG4.

**5.** Composition destinée à une utilisation selon l'une quelconque des revendications 1, 3 et 4, **caractérisée en ce que** ledit anticorps est un anticorps monoclonal IgG2 humain.

**6.** Composition destinée à une utilisation selon l'une quelconque des revendications 1-5, **caractérisée en ce que** l'anticorps ou un fragment de celui-ci inhibe la fixation d'IL-17A audit IL-17RA.

**7.** Composition destinée à une utilisation selon la revendication 6, **caractérisée en ce que** le patient possède environ 7% ou moins, environ 5% ou moins, environ 3% ou moins, ou environ 1% ou moins, de surface corporelle affectée par le psoriasis.

**8.** Composition destinée à une utilisation selon l'une quelconque des revendications 1-3, **caractérisée en ce que** le patient souffre de psoriasis en plaques, de psoriasis pustuleux ou de psoriasis érythrodermique.

**9.** Composition destinée à une utilisation selon la revendication 8, **caractérisée en ce que** :

(a) le patient possède :

(i) un score NAPSI modifié d'au moins 2 ou 3 sur un ou plusieurs ongles, et/ou
(ii) un score de Surface de Cuir Chevelu (SSA) d'au moins 30%, et/ou
(iii) moins de 10% de surface corporelle affectée par le psoriasis et atteint de plus de psoriasis des ongles ou du cuir chevelu, et/ou
(iv) moins de 10% de surface corporelle affectée par un psoriasis modéré à grave, un psoriasis en plaques, un psoriasis pustuleux ou un psoriasis érythrodermique, et/ou

(b) la composition comprend une dose d'anticorps qui est de 70 mg, 140 mg, 210 mg ou 280 mg.

**10.** Composition destinée à une utilisation selon l'une quelconque des revendications 1-9, **caractérisée en ce que** le traitement comprend l'administration de la composition avec un deuxième traitement.

**11.** Composition destinée à une utilisation selon la revendication 10, **caractérisée en ce que** ledit deuxième traitement est administré préalablement, simultanément ou ultérieurement à l'administration dudit médicament ou de ladite composition comprenant ledit anticorps.

**12.** Composition destinée à une utilisation selon la revendication 10 ou la revendication 11, **caractérisée en ce que** ledit deuxième traitement est un traitement topique, éventuellement où le traitement topique est choisi dans le groupe constitué par le fluorouracile, le dithranol, le tazarotène, la cyclosporine, les inhibiteurs de calcineurine, la triamcinolone, le fluocinonide, les stéroïdes topiques, la vitamine D3, les analogues de vitamine D3, le dipropionate de bétaméthasone, le valérate de bétaméthasone, le calcipotriol, le clobétasol, le Xamiol, le Daivobet, le goudron de houille, l'urée, les corticostéroïdes, les rétinoïdes, l'anthraline, le méthotrexate topique, les substances kératolytiques, l'acide salicylique, le tofacitinib, l'aprémilast, les inhibiteurs topiques de JAK ou une combinaison de ceux-ci.

**13.** Composition destinée à une utilisation selon l'une quelconque des revendications 10-12, **caractérisée en ce que** le deuxième traitement est choisi dans le groupe constitué par les rétinoïdes, l'acitrétine, la cyclosporine, le méthotrexate, l'aprémilast, le tofacitinib, les inhibiteurs oraux de JAK, les inhibiteurs oraux de PI3 kinase, les inhibiteurs oraux de MAP kinase, le Fumaderm, les fumarates, le fumarate de diméthyle, la sulfasalazine, le léflunomide, les inhibiteurs de calcineurine, l'azathioprine, la thioguanine, l'hydroxyurée, l'hydroxychloroquine, la sulfasalazine, les substances antifongiques ou une combinaison de ceux-ci.

**14.** Composition destinée à une utilisation selon l'une quelconque des revendications 10-13, **caractérisée en ce que** le deuxième traitement est :

(a) un anticorps ou une protéine chimère spécifique du TNF, de l'IL-17, de l'IL-12/23 ou de l'IL-23, éventuellement où l'anticorps ou la protéine chimère est l'infliximab, l'adalimumab, l'étanercept, l'aléfacept, l'ustékinumab, le sécukinumab, l'ixékizumab, le guselkumab, les substances antifongiques ou une combinaison de ceux-ci, et/ou
(b) choisi dans le groupe constitué par une photochimiothérapie à base de triamcinolone acétonide, une thérapie au laser, un laser à excimère, du psoralène oral/topique avec des UVA (PUV A), un laser à colorant puisé, une radiothérapie, une radiothérapie superficielle, une thérapie par faisceaux d'électrons, une thérapie à rayons de Bucky, un rasage du dermatome, un extrait d'aloé-vera, une thérapie par UV à bande étroite, une thérapie par UV ou une combinaison de ceux-ci.

**15.** Composition destinée à une utilisation selon l'une quelconque des revendications 1-14, **caractérisée en ce que** ladite composition comprend en outre un diluant pharmaceutiquement acceptable.

**16.** Composition destinée à une utilisation selon l'une quelconque des revendications 1-15, **caractérisée en ce que** ladite composition comprend une solution aqueuse d'un tampon à base d'acide glutamique, et où a) ledit tampon à base d'acide glutamique comprend une concentration en acide glutamique de 5-30 mM $\pm$ 0,2 mM ; b) ledit tampon à base d'acide glutamique comprend un pH de 4,5-5,2 $\pm$ 0,2 ; c) ladite formulation comprend en outre 2-4% de proline (p/v) et 0,005-0,02% (p/v) de polysorbate 20 et d) l'anticorps ou le fragment de celui-ci se trouve selon une concentration allant de 100 à 150 mg/ml.

**17.** Composition destinée à une utilisation selon la revendication 16, **caractérisée en ce que** la composition possède :

(a) une osmolarité allant de 275 à 325 Osm ; et/ou
(b) une viscosité allant de 5 à 7 cP à 25°C.

Study Design and Treatment Schema

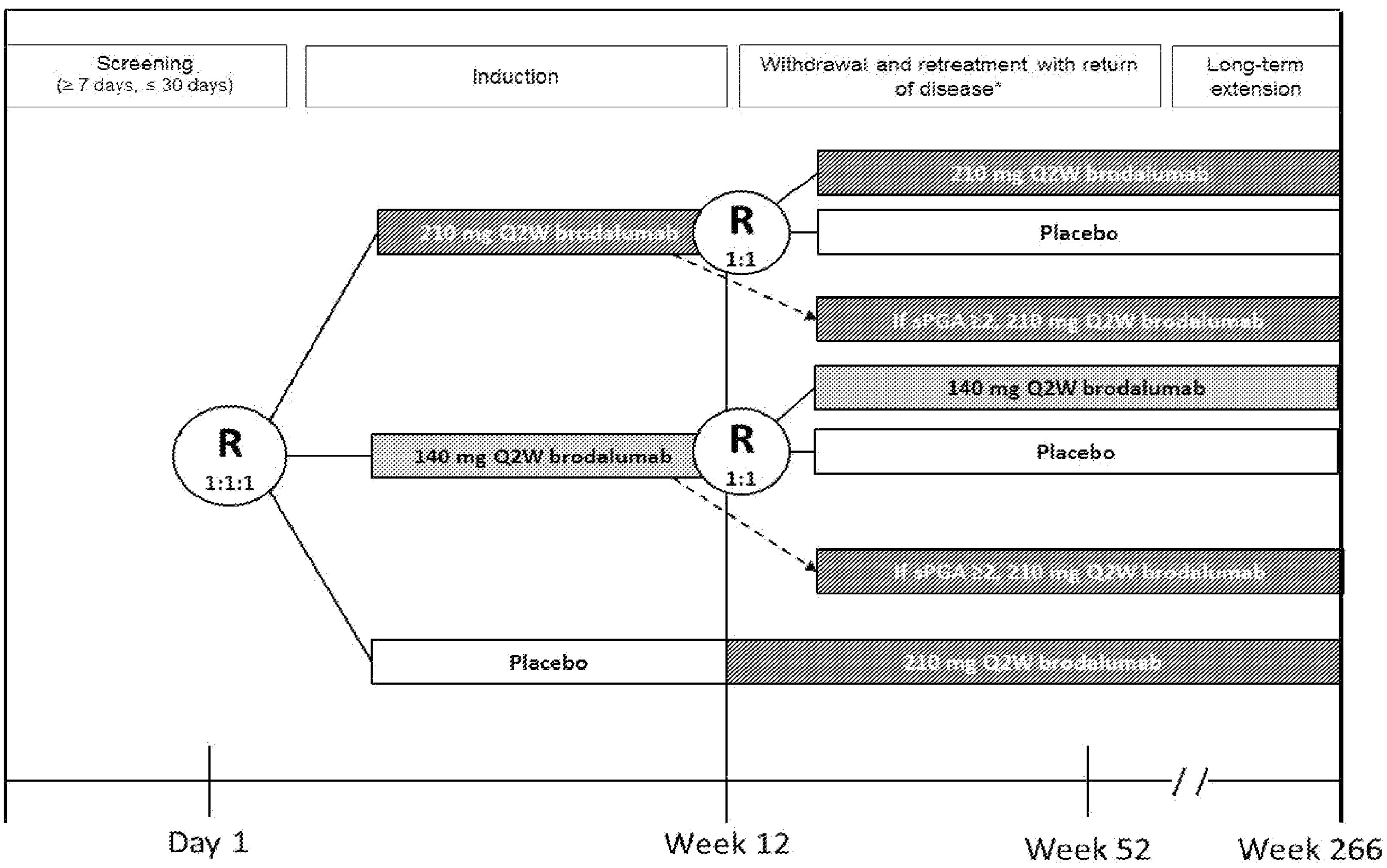


FIGURE 1

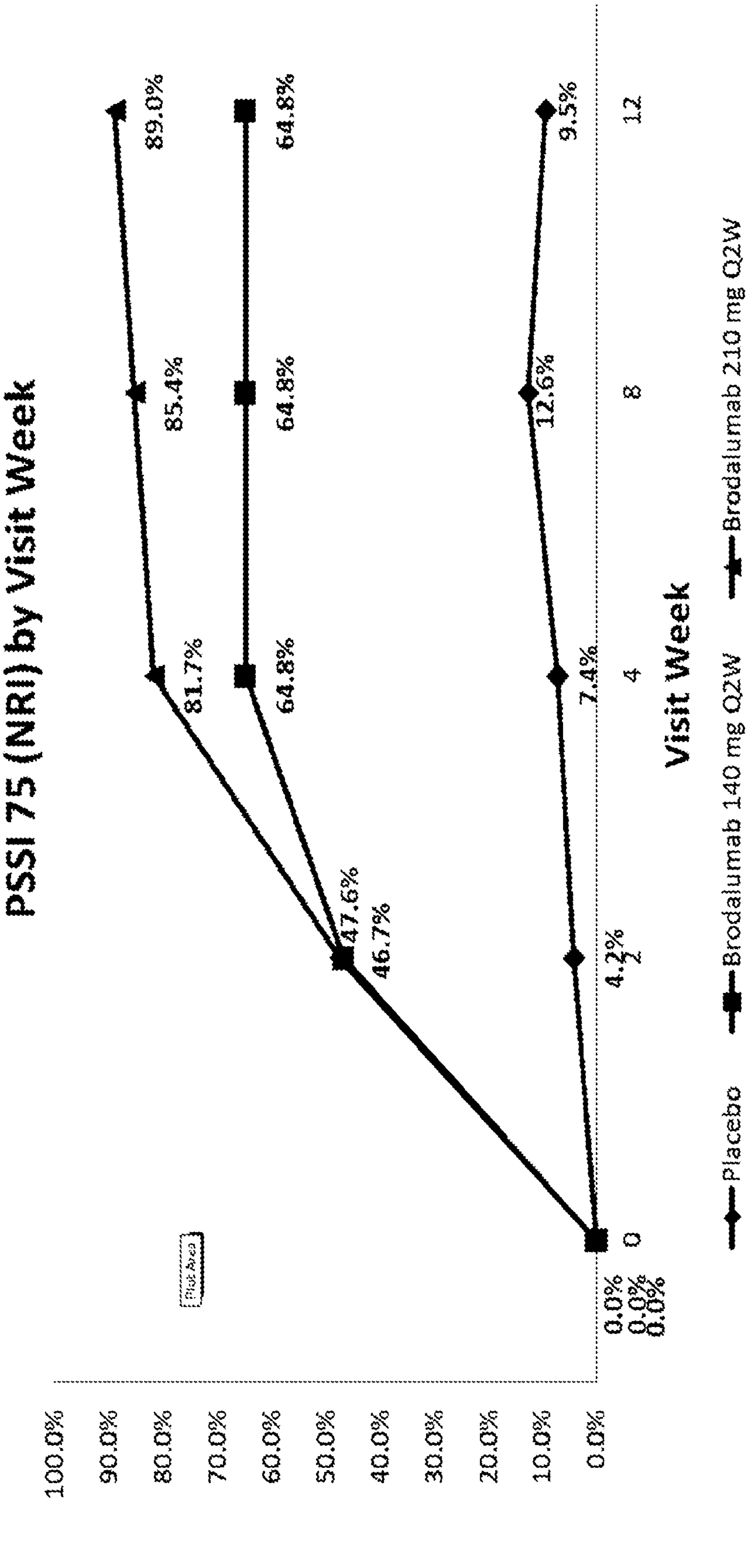
PSSI 75 (NRI) by Visit Week
100.0%
90.0%
80.0%
70.0%
60.0%
50.0%
40.0%
30.0%
20.0%
10.0%
0.0%
0.0%
0.0%
0.0%
46.7%
47.6%
4.2%
81.7%
64.8%
7.4%
85.4%
64.8%
12.6%
89.0%
64.8%
9.5%
0
2
4
8
12
Visit Week
Placebo
Brodalumab 140 mg Q2W
Brodalumab 210 mg Q2W

FIGURE 2

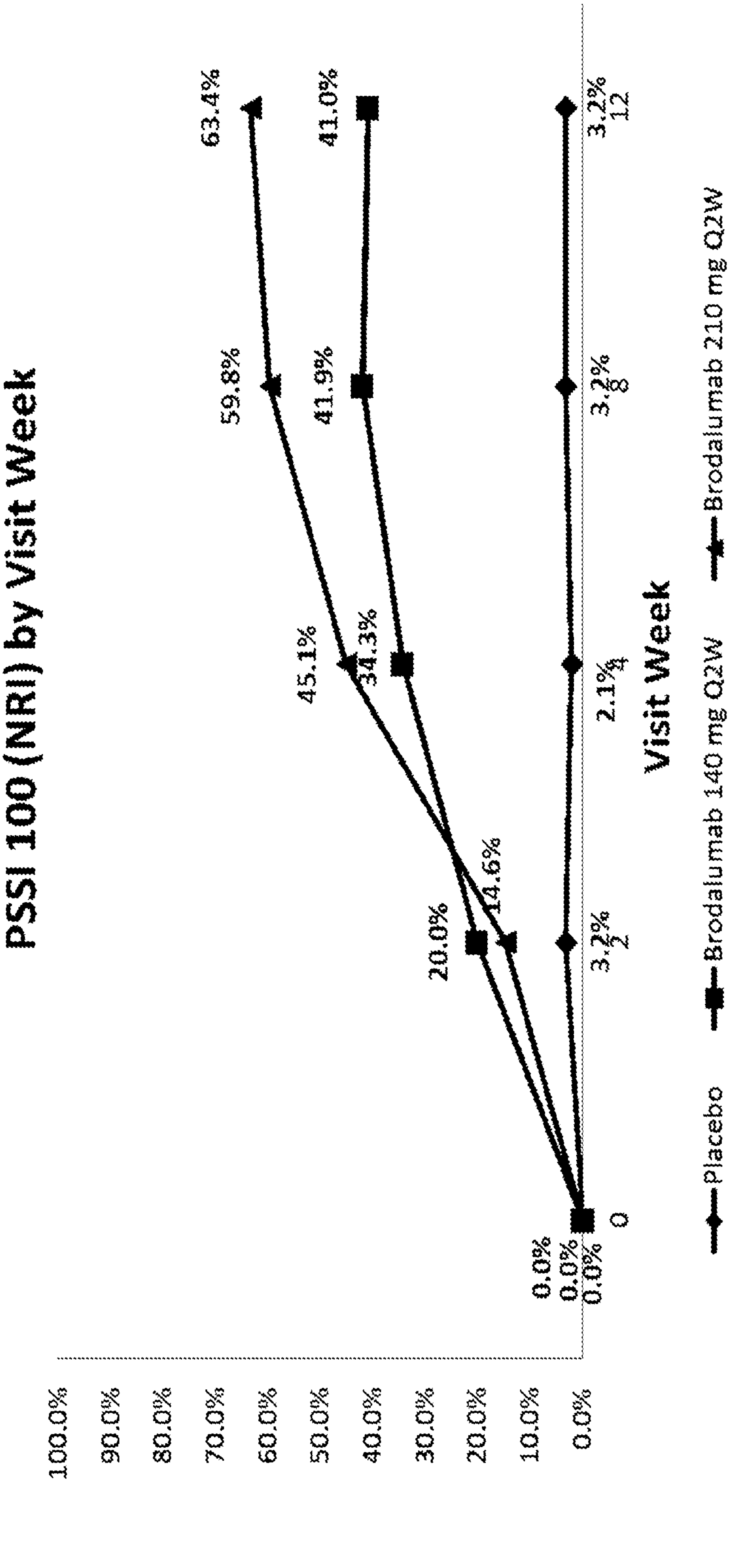
PSSI 100 (NRI) by Visit Week
100.0%
90.0%
80.0%
70.0%
60.0%
50.0%
40.0%
30.0%
20.0%
10.0%
0.0%
0.0%
0.0%
0.0%
20.0%
14.6%
3.2%
45.1%
34.3%
2.1%
59.8%
41.9%
3.2%
63.4%
41.0%
3.2%
0
2
4
8
12
Visit Week
Placebo
Brodalumab 140 mg Q2W
Brodalumab 210 mg Q2W

FIGURE 3

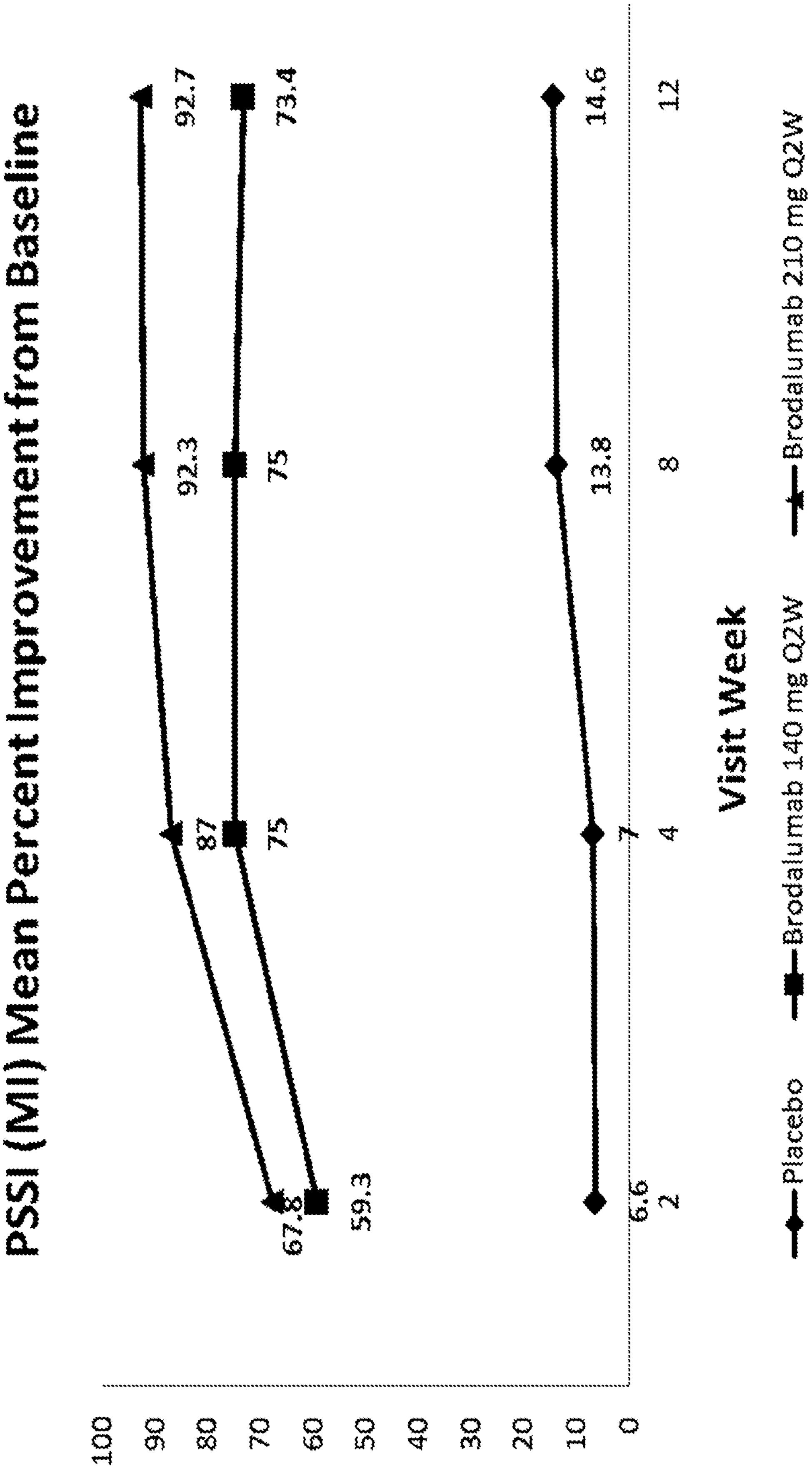
PSSI (MI) Mean Percent Improvement from Baseline
100
90
80
70
60
50
40
30
20
10
0
2
4
8
12
Visit Week
67.8
59.3
6.6
87
75
7
92.3
75
13.8
92.7
73.4
14.6
Placebo
Brodalumab 140 mg Q2W
Brodalumab 210 mg Q2W

FIGURE 4

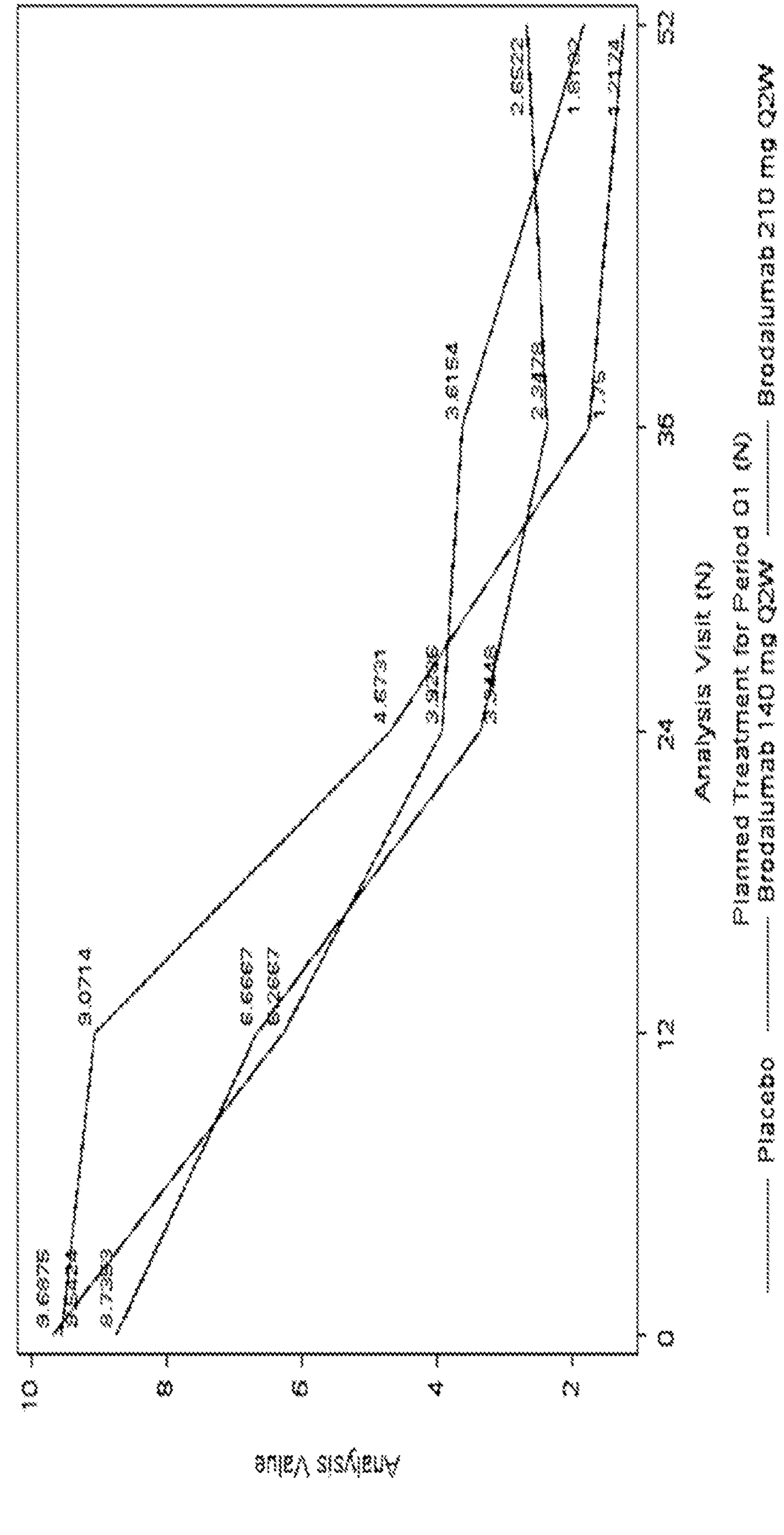
Analysis Value
10
8
6
4
2
0
12
24
36
52
9.6875
8.7353
9.0714
6.6667
4.6731
3.6154
1.75
2.6522
Analysis Visit (N)
Planned Treatment for Period 01 (N)
Placebo
Brodalumab 140 mg Q2W
Brodalumab 210 mg Q2W

FIGURE 5

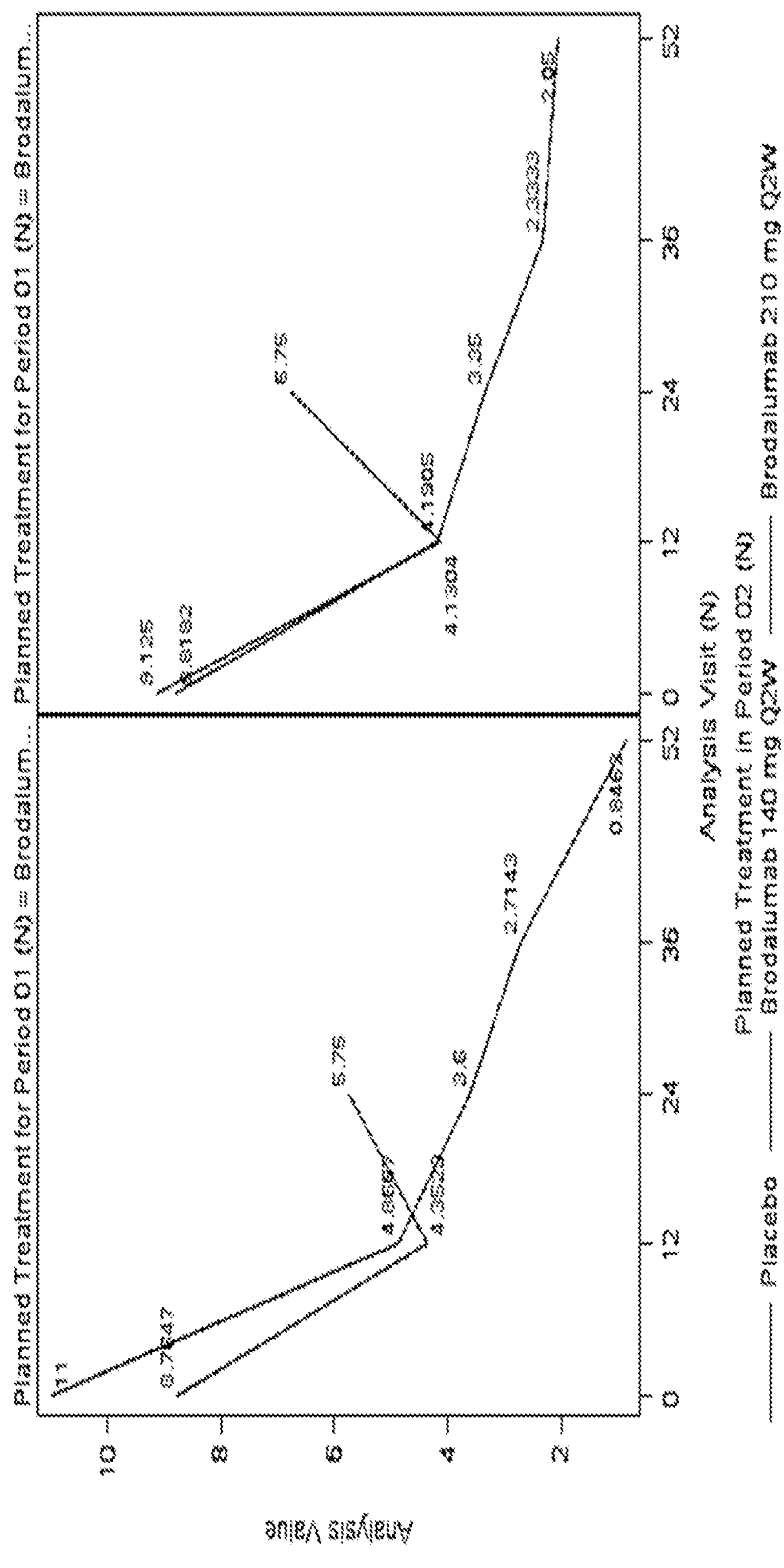

Planned Treatment for Period 01 (N) = Brodalum...
Planned Treatment for Period 01 (N) = Brodalum...
Analysis Value
10
8
6
4
2
0
12
24
36
52
11
8.7647
4.8667
4.3529
5.75
3.6
2.7143
0.8467
9.125
8.8182
4.1304
4.1905
6.75
3.35
2.3333
2.05
Analysis Visit (N)
Planned Treatment in Period 02 (N)
Placebo
Brodalumab 140 mg Q2W
Brodalumab 210 mg Q2W


FIGURE 6

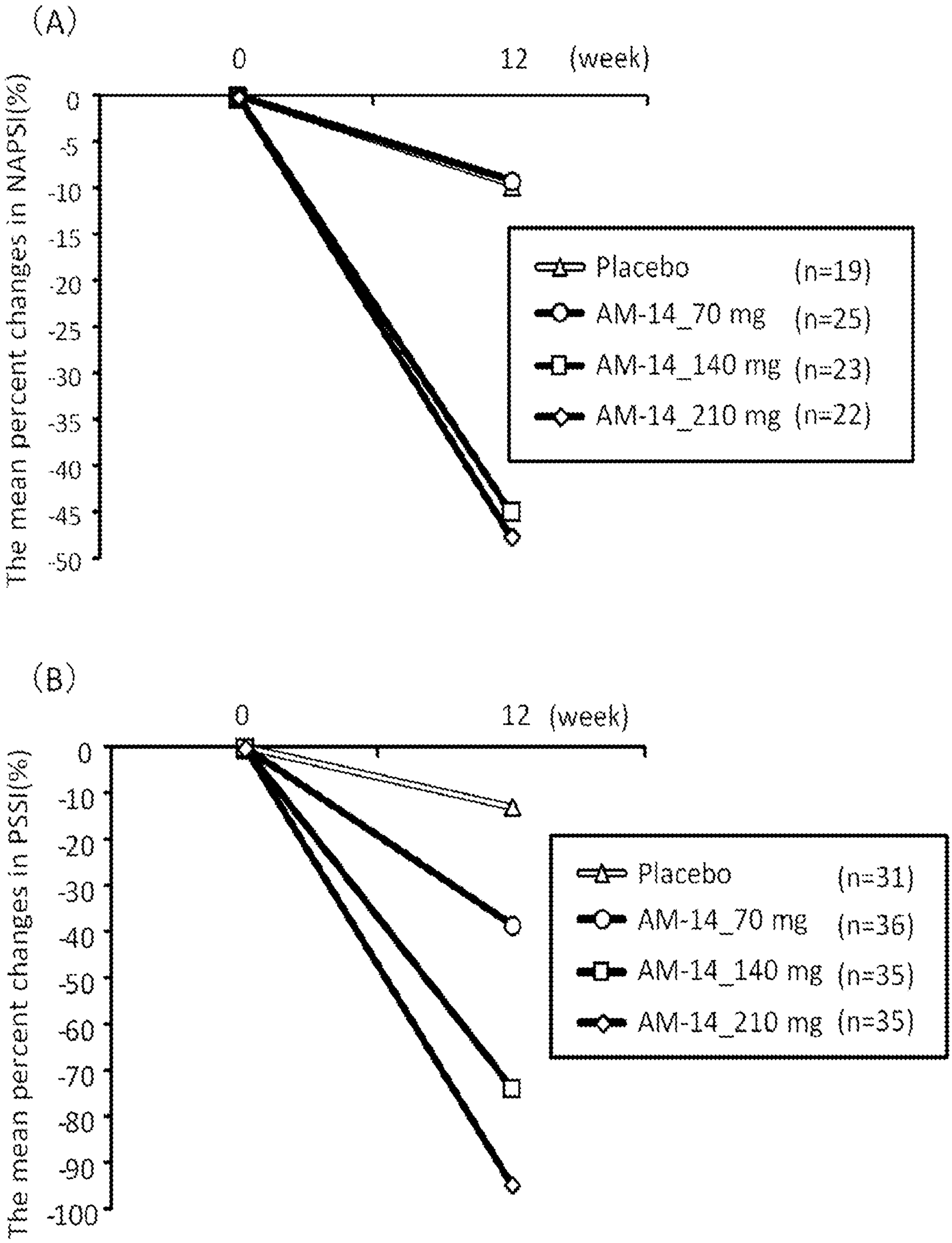
(A)
0
12 (week)
The mean percent changes in NAPSI(%)
0
-5
-10
-15
-20
-25
-30
-35
-40
-45
-50
Placebo (n=19)
AM-14_70 mg (n=25)
AM-14_140 mg (n=23)
AM-14_210 mg (n=22)
(B)
0
12 (week)
The mean percent changes in PSSI(%)
0
-10
-20
-30
-40
-50
-60
-70
-80
-90
-100
Placebo (n=31)
AM-14_70 mg (n=36)
AM-14_140 mg (n=35)
AM-14_210 mg (n=35)

FIGURE 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011088120 A
- US 6072033 A
- US 7767206 B
- WO 9211018 A
- US 9209965 W
- WO 9413804 A
- US 4946778 A
- EP 308378 A
- EP 422339 A
- GB 2218101 A
- EP 393438 A
- WO 9013575 A
- EP 398327 A
- EP 412486 A
- WO 9103553 A
- EP 418014 A
- JP 3127800 A
- EP 433900 A
- US 5136021 A
- GB 2246569 A
- EP 464533 A
- WO 9201002 A
- WO 9213095 A
- WO 9216221 A
- EP 512528 A
- EP 526905 A
- WO 9307863 A
- EP 568928 A
- WO 9321946 A
- WO 9319777 A
- EP 417563 A
- WO 9406476 A
- WO 98001555 A
- WO 9534326 A
- US 5116964 A
- WO 8909622 A
- WO 9116437 A
- EP 315062 A
- US 20130022621 A
- WO 06138181 A2
- US 61972638 B
- US 62031850 B
- US 62041879 B


### Non-patent literature cited in the description


- **HERMANNS-LE et al.** *J. Biomed. Biotech. 2012,* 2012, 1-6
- **KIRCIK ; KUMAR.** *J. Drugs Dermolog,* 2010, vol. 9, 101-105
- **WOZEL.** *Clin. Derm.,* 2008, vol. 26, 448-459
- **YAO et al.** *Immunity,* 1995, vol. 3, 811-821
- **KOLLS ; LINDEN.** *Immunity,* 2004, vol. 21, 467-476
- **LI et al.** *Huazhong Univ. Sci. Technolog. Med. Sci.,* 2004, vol. 24, 294-296
- **FUJINO et al.** *Gut,* 2003, vol. 52, 65-70
- **KAUFFMAN et al.** *J. Invest. Dermatol.,* 2004, vol. 123, 1037-1044
- **MANNON et al.** *N. Engl. J Med.,* 2004, vol. 351, 2069-2079
- **MATUSEVICIUS et al.** *Mult Scler,* 1999, vol. 5, 101-104
- **LINDEN et al.** *Eur Respir J.,* May 2000, vol. 15 (5), 973-7
- **MOLET et al.** *J. Allergy Clin. Immunol.,* 2001, vol. 108, 430-438
- **ODA et al.** *American J. Resp. Crit. Care Medicine,* 15 January 2006
- **NUMASAKI et al.** *Immunol Lett.,* 2004, vol. 95, 97-104
- **LEONARDI et al.** *NEJM,* 2012, vol. 366 (13), 1190-1199
- **GUDJONSSON et al.** *J Invest Dermatol.,* 2015, vol. 118 (2), 362-5
- **RICH ; SCHER.** *J. AM. Acad. Dermalol.,* 2003, vol. 49 (2), 206-12
- **SAMBROOK et al.** Molecular Cloning: A Laboratory Manual. Cold Spring Harbor Laboratory Press, 2001
- **JONES.** *Nature,* 1986, vol. 321, 522-525
- **VERHOEYEN et al.** *Science,* 1988, vol. 239, 1534-1536
- **ROQUE et al.** *Biotechnol. Prog.,* 2004, vol. 20, 639-654
- **HOLLIGER ; WINTER.** *Current Opinion Biotechnol.,* 1993, vol. 4, 446-449
- **HU et al.** *Cancer Res.,* 1996, vol. 56, 3055-3061
- **WARD et al.** *Nature,* 1989, vol. 341, 544-546
- **BIRD et al.** *Science,* 1988, vol. 242, 423-426

- **HUSTON et al.** *Proc. Natl. Acad. Sci. U.S.A.,* 1988, vol. 85, 5879-5883
- **TOMLINSON.** *Methods Enzymol.,* 2000, vol. 326, 461-479
- **BOLLIGER et al.** *Proc. Natl. Acad. Sci. U.S.A.,* 1993, vol. 90, 6444-6448
- **REITER et al.** *Nature Biotech.,* 1996, vol. 14, 1239-1245
- **KORTT et al.** *Prot. Eng.,* 1997, vol. 10, 423
- **KORTT et al.** *Biomol. Eng.,* 2001, vol. 18, 95-108
- **KRIANGKUM et al.** *Biomol. Eng.,* 2001, vol. 18, 31-40
- **BIRD.** *Science,* 1988, vol. 242, 423
- **HUSTON et al.** *Proc. Natl. Acad. Sci. USA,* 1988, vol. 85, 5879
- **WARD et al.** *Nature,* 1989, vol. 334, 544
- **DE GRAAF et al.** *Methods Mol Biol.,* 2002, vol. 178, 379-87
- **SIMON et al.** *Proc. Natl. Acad. Sci. U.S.A.,* 1992, vol. 89, 9367
- The Merck Manual of Diagnosis and Therapy. Merck, Sharp & Dohme Research Laboratories, Merck & Co, 1992
- **NEVE et al.** *Cytokine,* 1996, vol. 8 (5), 365-370
- **LIVINGSTON et al.** Identification and Characterization of Synthetic Small Molecule Macrocycle Antagonists of Human IL17A. *ACR Annual Meeting,* 09 November 2012
- **GOODMAN ; GILMAN.** The Pharmacological Basis of Therapeutics. MacMillan, 1985
- REMINGTON'S PHARMACEUTICAL SCIENCES. Mack Publishing Company, 1990
- **RICH et al.** *J Am Acad Dermatol,* vol. 49 (2), 206-212
- **LEONARDI et al.** *J Engl J Med,* 2012, vol. 366, 1190-9

SEQUENCE LISTING

<110> KIRIN-AMGEN, INC.

<120> METHODSW OF TREATING NAIL AND SCALP PSORIASIS

<130> 32053/48326A

<150> US 61/972,638
<151> 2014-03-31

<150> US 62/031,850
<151> 2014-07-30

<150> US 62/ 041,879
<151> 2014-08-26

<160> 13

<170> PatentIn version 3.5

<210> 1
<211> 11
<212> PRT
<213> Homo sapiens

<400> 1

```
Arg Ala Ser Gln Ser Val Ser Ser Asn Leu Ala
1               5                   10
```

<210> 2
<211> 7
<212> PRT
<213> Homo sapiens

<400> 2

```
Asp Ala Ser Thr Arg Ala Thr
1               5
```

<210> 3
<211> 9
<212> **PRT**
<213> Homo sapiens

<400> 3

```
Gln Gln Tyr Asp Asn Trp Pro Leu Thr
1               5
```

<210> 4
<211> 5
<212> PRT
<213> Homo sapiens

<400> 4

Arg Tyr Gly Ile Ser
1 5

<210> 5
<211> 17
<212> PRT
<213> Homo sapiens

<400> 5

Trp Ile Ser Thr Tyr Ser Gly Asn Thr Asn Tyr Ala Gln Lys Leu Gln
1 5 10 15

Gly

<210> 6
<211> 7
<212> PRT
<213> Homo sapiens

<400> 6

Arg Gln Leu Tyr Phe Asp Tyr
1 5

<210> 7
<211> 107
<212> PRT
<213> Homo sapiens

<400> 7

```
Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Phe Gln Gln Lys Pro Gly Gln Ala Pro Arg Pro Leu Ile
        35                  40                  45

Tyr Asp Ala Ser Thr Arg Ala Thr Gly Val Pro Ala Arg Phe Ser Gly
    50                  55                  60

Ser Gly Ser Gly Thr Asp Phe Thr Leu Thr Ile Ser Ser Leu Gln Ser
65                  70                  75                  80

Glu Asp Phe Ala Val Tyr Tyr Cys Gln Gln Tyr Asp Asn Trp Pro Leu
                85                  90                  95

Thr Phe Gly Gly Gly Thr Lys Val Glu Ile Lys
            100                 105
```

<210> 8
<211> 116
<212> PRT
<213> Homo sapiens

<400> 8

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Arg Tyr
            20                  25                  30

Gly Ile Ser Trp Val Arg Gln Ala Pro Gly Gln Gly Leu Glu Trp Met
        35                  40                  45

Gly Trp Ile Ser Thr Tyr Ser Gly Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Arg Gln Leu Tyr Phe Asp Tyr Trp Gly Gln Gly Thr Leu Val
            100                 105                 110

Thr Val Ser Ser
        115

<210> 9
<211> 214
<212> PRT
<213> Homo sapiens

<400> 9

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Phe Gln Gln Lys Pro Gly Gln Ala Pro Arg Pro Leu Ile
        35                  40                  45

Tyr Asp Ala Ser Thr Arg Ala Thr Gly Val Pro Ala Arg Phe Ser Gly
    50                  55                  60

Ser Gly Ser Gly Thr Asp Phe Thr Leu Thr Ile Ser Ser Leu Gln Ser

```
65                  70                  75                  80

Glu Asp Phe Ala Val Tyr Tyr Cys Gln Gln Tyr Asp Asn Trp Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 10
<211> 461
<212> PRT
<213> Homo sapiens

<400> 10

```
Met Glu Trp Thr Trp Arg Val Leu Phe Leu Val Ala Ala Ala Thr Gly
1               5                   10                  15

Ala His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Arg Tyr Gly Ile Ser Trp Val Arg Gln Ala Pro Gly Gln Gly Leu
    50                  55                  60

Glu Trp Met Gly Trp Ile Ser Thr Tyr Ser Gly Asn Thr Asn Tyr Ala
```

65 70 75 80

Gln Lys Leu Gln Gly Arg Val Thr Met Thr Thr Asp Thr Ser Thr Ser
85 90 95

Thr Ala Tyr Met Glu Leu Arg Ser Leu Arg Ser Asp Asp Thr Ala Val
100 105 110

Tyr Tyr Cys Ala Arg Arg Gln Leu Tyr Phe Asp Tyr Trp Gly Gln Gly
115 120 125

Thr Leu Val Thr Val Ser Ser Ala Ser Thr Lys Gly Pro Ser Val Phe
130 135 140

Pro Leu Ala Pro Cys Ser Arg Ser Thr Ser Glu Ser Thr Ala Ala Leu
145 150 155 160

Gly Cys Leu Val Lys Asp Tyr Phe Pro Glu Pro Val Thr Val Ser Trp
165 170 175

Asn Ser Gly Ala Leu Thr Ser Gly Val His Thr Phe Pro Ala Val Leu
180 185 190

Gln Ser Ser Gly Leu Tyr Ser Leu Ser Ser Val Val Thr Val Pro Ser
195 200 205

Ser Asn Phe Gly Thr Gln Thr Tyr Thr Cys Asn Val Asp His Lys Pro
210 215 220

Ser Asn Thr Lys Val Asp Lys Thr Val Glu Arg Lys Cys Cys Val Glu
225 230 235 240

Cys Pro Pro Cys Pro Ala Pro Pro Val Ala Gly Pro Ser Val Phe Leu
245 250 255

Phe Pro Pro Lys Pro Lys Asp Thr Leu Met Ile Ser Arg Thr Pro Glu
260 265 270

Val Thr Cys Val Val Val Asp Val Ser His Glu Asp Pro Glu Val Gln
275 280 285

Phe Asn Trp Tyr Val Asp Gly Val Glu Val His Asn Ala Lys Thr Lys
290 295 300

Pro Arg Glu Glu Gln Phe Asn Ser Thr Phe Arg Val Val Ser Val Leu
305 310 315 320

Thr Val Val His Gln Asp Trp Leu Asn Gly Lys Glu Tyr Lys Cys Lys
325 330 335

Val Ser Asn Lys Gly Leu Pro Ala Pro Ile Glu Lys Thr Ile Ser Lys
340 345 350

Thr Lys Gly Gln Pro Arg Glu Pro Gln Val Tyr Thr Leu Pro Pro Ser
355 360 365

Arg Glu Glu Met Thr Lys Asn Gln Val Ser Leu Thr Cys Leu Val Lys
370 375 380

Gly Phe Tyr Pro Ser Asp Ile Ala Val Glu Trp Glu Ser Asn Gly Gln
385 390 395 400

Pro Glu Asn Asn Tyr Lys Thr Thr Pro Pro Met Leu Asp Ser Asp Gly
405 410 415

Ser Phe Phe Leu Tyr Ser Lys Leu Thr Val Asp Lys Ser Arg Trp Gln
420 425 430

Gln Gly Asn Val Phe Ser Cys Ser Val Met His Glu Ala Leu His Asn
435 440 445

His Tyr Thr Gln Lys Ser Leu Ser Leu Ser Pro Gly Lys
450 455 460

<210> 11
<211> 741
<212> DNA
<213> Homo sapiens

<400> 11

gtcgacgttt aaacgccgcc accatggaag cgccggcgca gcttctcttc ctcctgctac 60

tctggctccc agataccact ggagaaatag tgatgacgca gtctccagcc accctgtctg 120

tgtctcctgg ggaaagagcc accctctcct gcagggccag tcagagtgtt agcagcaact 180

tagcctggtt ccagcagaaa cctggccagg ctcccaggcc cctcatctat gatgcatcca 240

ccagggccac tggtgtccca gccaggttca gtggcagtgg gtctgggaca gacttcactc 300

tcaccatcag cagcctgcag tctgaagatt ttgcagttta ttactgtcag cagtatgata 360

actggccgct cactttcggc ggagggacca aggtggagat caaacgtacg gtggctgcac 420

catctgtctt catcttcccg ccatctgatg agcagttgaa atctggaact gcctctgttg 480

tgtgcctgct gaataacttc tatcccagag aggccaaagt acagtggaag gtggataacg 540

ccctccaatc gggtaactcc caggagagtg tcacagagca ggacagcaag gacagcacct 600

acagcctcag cagcaccctg acgctgagca aagcagacta cgagaaacac aaagtctacg 660

cctgcgaagt cacccatcag ggcctgagct cgcccgtcac aaagagcttc aacaggggag 720

agtgttagga tccgcggccg c 741

<210> 12
<211> 1409
<212> DNA
<213> Homo sapiens

<400> 12

gtcgacgccg ccaccatgga gtggacctgg agggtccttt tcttggtggc agcagcaaca 60
ggtgcccact cccaggttca gctggtgcag tctggagctg aggtgaagaa gcctggggcc 120
tcagtgaagg tctcctgcaa ggcttctggt tacaccttta ccagatatgg tatcagctgg 180
gtgcgacagg cccctggaca agggcttgag tggatgggat ggatcagcac ttacagtggt 240
aacacaaact atgcacagaa gctccagggc agagtcacca tgaccacaga cacatccacg 300
agcacagcct acatggagct gaggagcctg agatctgacg acacggccgt gtattactgt 360
gcgagacggc agctttactt tgactactgg ggccagggaa ccctggtcac cgtctcctca 420
gctagcacca agggcccatc ggtcttcccc ctggcgccct gctccaggag cacctccgag 480
agcacagcgg ccctgggctg cctggtcaag gactacttcc ccgaaccggt gacggtgtcg 540
tggaactcag gcgctctgac cagcggcgtg cacaccttcc cagctgtcct acagtcctca 600
ggactctact ccctcagcag cgtggtgacc gtgccctcca gcaacttcgg cacccagacc 660
tacacctgca acgtagatca caagcccagc aacaccaagg tggacaagac agttgagcgc 720
aaatgttgtg tcgagtgccc accgtgccca gcaccacctg tggcaggacc gtcagtcttc 780
ctcttccccc caaaacccaa ggacaccctc atgatctccc ggacccctga ggtcacgtgc 840
gtggtggtgg acgtgagcca cgaagacccc gaggtccagt tcaactggta cgtggacggc 900
gtggaggtgc ataatgccaa gacaaagcca cgggaggagc agttcaacag cacgttccgt 960
gtggtcagcg tcctcaccgt tgtgcaccag gactggctga acggcaagga gtacaagtgc 1020
aaggtctcca acaaaggcct cccagccccc atcgagaaaa ccatctccaa aaccaaaggg 1080
cagccccgag aaccacaggt gtacaccctg cccccatccc gggaggagat gaccaagaac 1140
caggtcagcc tgacctgcct ggtcaaaggc ttctacccca gcgacatcgc cgtggagtgg 1200
gagagcaatg ggcagccgga gaacaactac aagaccacac ctcccatgct ggactccgac 1260
ggctccttct tcctctacag caagctcacc gtggacaaga gcaggtggca gcaggggaac 1320
gtcttctcat gctccgtgat gcatgaggct ctgcacaacc actacacgca gaagagcctc 1380
tccctgtctc cgggtaaatg agcggccgc 1409

<210> 13
<211> 866
<212> PRT
<213> Homo sapiens

<400> 13

```
Met Gly Ala Ala Arg Ser Pro Pro Ser Ala Val Pro Gly Pro Leu Leu
1               5                   10                  15

Gly Leu Leu Leu Leu Leu Leu Gly Val Leu Ala Pro Gly Gly Ala Ser
            20                  25                  30

Leu Arg Leu Leu Asp His Arg Ala Leu Val Cys Ser Gln Pro Gly Leu
        35                  40                  45

Asn Cys Thr Val Lys Asn Ser Thr Cys Leu Asp Asp Ser Trp Ile His
    50                  55                  60

Pro Arg Asn Leu Thr Pro Ser Ser Pro Lys Asp Leu Gln Ile Gln Leu
65                  70                  75                  80

His Phe Ala His Thr Gln Gln Gly Asp Leu Phe Pro Val Ala His Ile
                85                  90                  95

Glu Trp Thr Leu Gln Thr Asp Ala Ser Ile Leu Tyr Leu Glu Gly Ala
            100                 105                 110

Glu Leu Ser Val Leu Gln Leu Asn Thr Asn Glu Arg Leu Cys Val Arg
        115                 120                 125

Phe Glu Phe Leu Ser Lys Leu Arg His His His Arg Arg Trp Arg Phe
    130                 135                 140

Thr Phe Ser His Phe Val Val Asp Pro Asp Gln Glu Tyr Glu Val Thr
145                 150                 155                 160

Val His His Leu Pro Lys Pro Ile Pro Asp Gly Asp Pro Asn His Gln
                165                 170                 175

Ser Lys Asn Phe Leu Val Pro Asp Cys Glu His Ala Arg Met Lys Val
            180                 185                 190

Thr Thr Pro Cys Met Ser Ser Gly Ser Leu Trp Asp Pro Asn Ile Thr
        195                 200                 205

Val Glu Thr Leu Glu Ala His Gln Leu Arg Val Ser Phe Thr Leu Trp
    210                 215                 220

Asn Glu Ser Thr His Tyr Gln Ile Leu Leu Thr Ser Phe Pro His Met
225                 230                 235                 240
```

```
Glu Asn His Ser Cys Phe Glu His Met His His Ile Pro Ala Pro Arg
                245                 250                 255

Pro Glu Glu Phe His Gln Arg Ser Asn Val Thr Leu Thr Leu Arg Asn
            260                 265                 270

Leu Lys Gly Cys Cys Arg His Gln Val Gln Ile Gln Pro Phe Phe Ser
        275                 280                 285

Ser Cys Leu Asn Asp Cys Leu Arg His Ser Ala Thr Val Ser Cys Pro
    290                 295                 300

Glu Met Pro Asp Thr Pro Glu Pro Ile Pro Asp Tyr Met Pro Leu Trp
305                 310                 315                 320

Val Tyr Trp Phe Ile Thr Gly Ile Ser Ile Leu Leu Val Gly Ser Val
                325                 330                 335

Ile Leu Leu Ile Val Cys Met Thr Trp Arg Leu Ala Gly Pro Gly Ser
            340                 345                 350

Glu Lys Tyr Ser Asp Asp Thr Lys Tyr Thr Asp Gly Leu Pro Ala Ala
        355                 360                 365

Asp Leu Ile Pro Pro Pro Leu Lys Pro Arg Lys Val Trp Ile Ile Tyr
    370                 375                 380

Ser Ala Asp His Pro Leu Tyr Val Asp Val Val Leu Lys Phe Ala Gln
385                 390                 395                 400

Phe Leu Leu Thr Ala Cys Gly Thr Glu Val Ala Leu Asp Leu Leu Glu
                405                 410                 415

Glu Gln Ala Ile Ser Glu Ala Gly Val Met Thr Trp Val Gly Arg Gln
            420                 425                 430

Lys Gln Glu Met Val Glu Ser Asn Ser Lys Ile Ile Val Leu Cys Ser
        435                 440                 445

Arg Gly Thr Arg Ala Lys Trp Gln Ala Leu Leu Gly Arg Gly Ala Pro
    450                 455                 460

Val Arg Leu Arg Cys Asp His Gly Lys Pro Val Gly Asp Leu Phe Thr
465                 470                 475                 480

Ala Ala Met Asn Met Ile Leu Pro Asp Phe Lys Arg Pro Ala Cys Phe
```

```
                485                 490                 495

Gly Thr Tyr Val Val Cys Tyr Phe Ser Glu Val Ser Cys Asp Gly Asp
            500                 505                 510

Val Pro Asp Leu Phe Gly Ala Ala Pro Arg Tyr Pro Leu Met Asp Arg
        515                 520                 525

Phe Glu Glu Val Tyr Phe Arg Ile Gln Asp Leu Glu Met Phe Gln Pro
    530                 535                 540

Gly Arg Met His Arg Val Gly Glu Leu Ser Gly Asp Asn Tyr Leu Arg
545                 550                 555                 560

Ser Pro Gly Gly Arg Gln Leu Arg Ala Ala Leu Asp Arg Phe Arg Asp
                565                 570                 575

Trp Gln Val Arg Cys Pro Asp Trp Phe Glu Cys Glu Asn Leu Tyr Ser
            580                 585                 590

Ala Asp Asp Gln Asp Ala Pro Ser Leu Asp Glu Glu Val Phe Glu Glu
        595                 600                 605

Pro Leu Leu Pro Pro Gly Thr Gly Ile Val Lys Arg Ala Pro Leu Val
    610                 615                 620

Arg Glu Pro Gly Ser Gln Ala Cys Leu Ala Ile Asp Pro Leu Val Gly
625                 630                 635                 640

Glu Glu Gly Gly Ala Ala Val Ala Lys Leu Glu Pro His Leu Gln Pro
                645                 650                 655

Arg Gly Gln Pro Ala Pro Gln Pro Leu His Thr Leu Val Leu Ala Ala
            660                 665                 670

Glu Glu Gly Ala Leu Val Ala Ala Val Glu Pro Gly Pro Leu Ala Asp
        675                 680                 685

Gly Ala Ala Val Arg Leu Ala Leu Ala Gly Glu Gly Glu Ala Cys Pro
    690                 695                 700

Leu Leu Gly Ser Pro Gly Ala Gly Arg Asn Ser Val Leu Phe Leu Pro
705                 710                 715                 720

Val Asp Pro Glu Asp Ser Pro Leu Gly Ser Ser Thr Pro Met Ala Ser
                725                 730                 735
```

```
Pro Asp Leu Leu Pro Glu Asp Val Arg Glu His Leu Glu Gly Leu Met
            740                 745                 750

Leu Ser Leu Phe Glu Gln Ser Leu Ser Cys Gln Ala Gln Gly Gly Cys
        755                 760                 765

Ser Arg Pro Ala Met Val Leu Thr Asp Pro His Thr Pro Tyr Glu Glu
    770                 775                 780

Glu Gln Arg Gln Ser Val Gln Ser Asp Gln Gly Tyr Ile Ser Arg Ser
785                 790                 795                 800

Ser Pro Gln Pro Pro Glu Gly Leu Thr Glu Met Glu Glu Glu Glu Glu
                805                 810                 815

Glu Glu Gln Asp Pro Gly Lys Pro Ala Leu Pro Leu Ser Pro Glu Asp
            820                 825                 830

Leu Glu Ser Leu Arg Ser Leu Gln Arg Gln Leu Leu Phe Arg Gln Leu
        835                 840                 845

Gln Lys Asn Ser Gly Trp Asp Thr Met Gly Ser Glu Ser Glu Gly Pro
    850                 855                 860

Ser Ala
865
```